# EUROPEAN PATENT SPECIFICATION

(11) **EP 2 241 284 B1**
(45) Date of publication and mention of the grant of the patent: **19.09.2012**
(21) Application number: 09157975.5
(22) Date of filing: 15.04.2009
(51) Int. Cl.: A61F 2/01, A61F 2/82, A61F 2/00, A61F 2/02, A61F 2/84

(54) **Intravasculature devices and balloons for use therewith**
Intravaskuläre Vorrichtungen und Ballons zur Verwendung damit
Dispositifs intra-vasculaires et ballons à utiliser avec ceux-ci

(43) Date of publication of application: 20.10.2010
(73) Proprietor: National University of Ireland, Galway, Galway (IE)
(72) Inventor: Murphy, Bruce, Galway (IE); Vard, John, Dublin (IE)
(74) Representative: Lane, Cathal Michael

(56) References cited:
- WO-A1-98/55047
- WO-A2-2009/027531
- FR-A2- 2 331 995
- US-A- 5 985 307
- US-A1- 2005 119 688
- US-A1- 2007 038 292

## Description

### Field of the Invention

The present invention relates to intravasculature devices and balloons for use therewith.

### Background to the Invention

Intravasculature devices including intravasculature filter devices are known.

In recent years, a debate has emerged between the use of carotid endarterectomy (CAE) or carotid angioplasty and stenting (CAS) to determine which is the most effective treatment for carotid disease. The high risk of embolisation that is associated with CAS means that the procedure is reliant on using embolic protection systems to capture the potentially harmful micro-debris that may escape downstream to cause blockages for example those resulting in a stroke etc. These systems are also routinely used during percutaneous treatment of saphenous vein graft (SVG) disease, and further applications are being found in the treatment of peripheral vascular disease (PVD) and in accompaniment for renal angioplasty procedures.

The systems currently on the market can be categorised as either distal embolic filters or distal/proximal occlusive balloons. The benefit of using a filter is that antegrade flow is maintained following deployment of the device. In contrast occlusive balloon devices function by stagnating flow in order to trap and prevent debris from travelling downstream. The problem with such devices is that some patients are intolerant to flow stagnation and in particular long durations thereof. On the other hand, the crossing profile of occlusive balloons is smaller than that achievable with current distal filter devices. For this reason, occlusive balloons are ideally suited to crossing highly occluded or delicate lesions.

Before protection can be initiated, a distal filter must first pass the occluded lesion. Routinely a delivery sheath is used and, following crossing of the lesion, the sheath is withdrawn to deploy the filter. Similarly during retrieval, a sheath (generally larger than the delivery sheath) is advanced to collapse and capture the filter, before it can be removed from the vasculature. The majority of devices employ this strategy during delivery and retrieval [for example the devices sold as FilterWire EZ® (Boston Scientific); Emboshield® (Abbott Laboratories); AngioGuard® (Cordis Group)].

A new generation of distal filter devices have started to address some of these issues by incorporating features that minimise the need for sheaths. For instance, the Rubicon® (Rubicon Medical) filter utilises an actuating wire instead of a sheath to keep the filter in its collapsed configuration during delivery, however a sheath is still required for retrieval. Another example is the FiberNet® (Lumen Biomedical), which like the Rubicon® filter only requires a sheath for retrieval. Unlike the present invention however, the FiberNet® device must be aspirated to ensure that any loosely collected debris is not lost from its fibrous mesh during collapse and retrieval.

In instances where the lesion is too difficult to cross the preferred strategy is to use a distal occlusive balloon (such as, Medtronic's GuardWire® system, or Kensey Nash's TriActiv® system) or in very extreme lesions where crossing is not possible a proximal occlusion balloon system. This choice is often due to the lower crossing profile of a distal balloon versus a filter, which is less likely to disturb the lesion during delivery.

The apposition of the filter to the vessel wall is considered to be another crucial factor in the performance of any distal embolic filter. Failure to conform to the vessel wall causes gaps to be introduced, through which debris can pass. Some of the factors that may complicate apposition include: the type of filter employed; the shape, size and tortuosity of the vessel; and/or the orientation of the guide wire within the vessel. For instance, the protection provided by traditional nitinol-framed filters is thought to be compromised in irregular shaped vessels (i.e. vessels with an elliptical shaped cross-section). In response to this, the FilterWire EZ® (Boston Scientific) and the Spider RX® (Ev3) incorporate a looped nitinol frame, which is thought to provide better apposition in tortuous elliptical shaped vessels. The FilterWire EZ® and the Spider RX® devices have been shown to perform well in regular shaped vessels, when compared against the more traditional filter types. A study by Finol *et al.* (Finol EA, Siewiorek GM, Scotti CM, Wholey MH, and Wholey MH. "Wall apposition assessment and performance comparison of distal protection filters." J. Endovasc. Ther. 2008; 15:177-185) observed that the FilterWire EZ® had the best overall average filtration rate, despite the RX Accunet® (Abbott Laboratories) showing the best overall wall apposition, yielding gaps of 0.075% of the vessel cross-sectional area. A similar study by Siewiorek *et al.* (Siewiorek GM, Wholey MH, and Finol EA. "In vitro performance assessment of distal protection devices for carotid artery stenting: effect of physiological anatomy on vascular resistance." J. Endovasc. Ther. 2007; 14:712-724) indicated that the Spider RX® performed best on account of superior wall appostion when compared against four other devices (FilterWire EZ®, RX Accunet®, AngioGuard XP®, and Emboshield®). Both studies refer to the fact that none of the devices used provided complete embolic protection (despite being tested in regular circular shaped vessel cross-sections) and that vessel wall apposition should be considered the primary design variable when guarding against distal embolisation.

The disclosed present invention, which involves the use of balloon technology, exceeds performance of the marketed distal/proximal occlusive protection systems, which use elastic balloons to occlude the vessel and stagnate antegrade blood flow. In principle, the use of balloon technology to anchor a device may be viewed as less harmful to the tissue of the vessel wall, due to greater surface area conformity and a more even loading of the vessel circumference, when compared against nitinol-framed devices, which have relatively few contact points with the vessel wall. The disclosed invention has the potential to operate in a host of different vessel shapes and sizes, thereby superseding the functionality of the majority of distal embolic filter devices and also providing an alternative to occlusive protection for irregular shaped, asymmetric lesions.

US Patent No. 4,723,549 describes a device comprises a collapsible filter. US Patent No. 4,794,928 describes an angioplasty balloon catheter with a trap on one end to catch debris. In certain embodiments trap is expanded by fluid. US Patent No. 5,053,008 describes a multi-sheathed catheter that has an umbrella assembly that has a meshwork for collecting debris. The umbrella is opened using a tubular balloon. A Doppler sensor and ultrasound are used to monitor the materials captured by the device.

US Patent No. 5,827,324 describes a filter system deployed by inflating a balloon. US Patent No. 5,947,995 describes a device with an inflatable cuff filter carried on a catheter. The cuff is cinched to catch blood clots, which are then removable from the body. A somewhat similar device is disclosed in US Patent No. 6,676,682**.** US Patent No. 6,053,932 describes a filter system on a guide wire for capturing emboli from blood. It describes a mesh structure which is expandable and thus deployable by an inflatable balloon. In certain embodiments the balloon has a thin elongate shape and is constructed of a shape-memory material configured to adapt a spiral shape. In certain embodiments the mesh is deployed by inflatable struts. A device which also uses an inflatable member in a spiral form is described in US Patent Publication No. 2001/0007947**.** US Patent Publication No. 2007/0038241 describes a similar arrangement with an inflatable frame of helical shape-memory material. In a somewhat similar arrangement US Patent Publication No. 2006/0047300 describes a filter device for filtering blood clots which is formed by an apical head and one or more inflatable legs connected to the head. Perfusion openings in a leg for slowly weeping anticoagulant or other therapeutic agent is disclosed. In one embodiment a secondary cavity is formed within the legs. In one embodiment the filter legs have a secondary cavity.

US Patent Publication No. 2002/0161390 describes a device with multiple filters. An expandable balloon opens out arms which deploy the filters for use. US Patent Publication No. 2002/0173819 utilises a jet to create a vacuum to suck debris in a body vessel such as an artery into a filter where it is trapped. In one embodiment a filter is used in conjunction with the device for example a mesh which comprises an arrangement with spokes extending from a central hub and attached to an outer collar of material. The collar and the spokes expand upon inflation to take up a deployed configuration of the filter. US Patent Publication No. 2004/0098026 describes a vascular filter constructed from a porous foam body.

US Patent No. 6,361,545 describes a filter device that can include a toroidal balloon or an expandable balloon. Alternatively it can include an inflatable hoop/struts arrangement. US Patent No. 6,491,712 describes a device for collecting debris flowing in an artery. The device is placed downstream from a plaque removal target site. It comprises a double walled balloon connected to a nitinol tube. A filter is secured to the balloon and is designed to collect debris from the blood which is generated when plaque is removed. The device is said to totally occlude the blood vessel, when inflated, thus ensuring all blood flows through the filter. US Patent No.s 6,506,203**,** 6,136,016 and US Patent Publication Nos. 2007/0173883 and 2007/0282368 describe alternative filter arrangements.

US Patent Publication No. 2006/0149314 relates to an implantable device which can be placed *in situ* and anchored in a target position within an artery. It has a membrane tube which acts as a filter for filtering blood. A balloon inside the membrane tube is used to expand or contract the membrane tube. US Patent No. 5,342,301 describes a thermoplastic balloon with multiple lumens therein.

French patent publication FR 2,331,995 describes a double wall sleeve made out of an elastic material such as natural rubber, where the space between the walls has a number of lumens defined therein. Each of the lumen is inflated by inserting a needle through the inner side wall into the lumen and providing inflation by sending a pressurised fluid through the needle.

US Patent No 5,985,307 describes a device for the local delivery of a substance into the vasculature. The device has an inflation chamber which comprises a number of interconnected inflation cells.

International Patent Publication WO 98/55047 describes an inflatable intraluminal stent in the form of a cuff and a filter that can be deployed in the inferior vena cava using the stent.

US Patent Publication No. 2007/0038292 describes a bioabsorbable stent that can be inflated. Pores of a predetermined size can be provided for the release of materials. Pre-filled pockets which hold drugs are also mentioned.

International Patent Publication WO 2009/027531 which has a common inventor with the present application discloses a minimally invasive intravascular device. US Patent Publication No. 2005/0119688 describes a filter assembly where the filter is opened out by a ring-shaped balloon.

### Summary of the Invention

The present invention relates to an expandable annular body which is expandable by inflation of a series of inflation lumen defined therein, the device having inflated and non-inflated states and inner and outer annular walls, the device being adapted so that inflationary pressure within the lumen moves the device from the non-inflated state toward the inflated state by radially outward expansion of both the outer and inner walls so that the annular body expands to form an annular structure with a central lumen defined by the inner wall and ballons which occupy the lumens. The device invention is capable of expanding to open a constricted vessel without substantial restriction of blood supply. It will be appreciated that the lumen are defined in the annular body itself (and not within the central lumen). The lumen can thus be considered to be defined in the body between the inner and outer walls. The central lumen defines blood-flow pathway through the device. The device of the invention is non-occlusive. Accordingly even if it is arranged for delivery by a carrier such as a guidewire and/or catheter it will be attached to the carrier in such a way as that the catheter does not occlude the central lumen, even if the carrier runs into the central lumen. Furthermore attachment of the device to a carrier will be arranged so as not to prevent radially outward expansion of both the outer and inner walls. In conventional carrier-delivered balloons an inner wall of the balloon remains static as it is fixed to the carrier. Such a conventional arrangement is occlusive.

In one embodiment the device of the invention is a double-walled elastic balloon, which is expandable from a collapsed state to an expanded state where the balloon contacts the arterial wall but maintains a central lumen at all times to allow blood flow through the central lumen. An external wall of the annular body contacts and seals against a vessel wall while an internal wall of the annular body defines a lumen.

The present invention has the potential to rival the crossing profile of balloon occlusion devices and in doing so neutralise the primary advantage they possess over current distal filter devices. The disclosed invention also allows for improved apposition to the vessel wall similar to that achievable by the elastic balloons of the occlusive devices.

The device of the present invention has at least three, preferably at least four, inflation lumen. This allows the device to expand in an even fashion about the central lumen. Generally the lumen will be sized and arranged to facilitate expansion to a desired shape. In one simple construction the inflation lumen are arranged substantially axially about the central lumen. For substantially even expansion for example to an open cylindrical shape it may be desirable to use lumen of substantially equal dimensions and to space those substantially equidistantly about the annular body.

In a further embodiment, the inner wall of the device of the present invention has a series of rebates or folds defined therein. This embodiment allows for ease of expansion and in particular ameliorates any bunching of the annular body about the inner diameter of the device. The rebates or folds are also desirably substantially evenly distributed about the annular body.

One embodiment of a device of the present invention wherein the expandable annular body is an annular double-walled balloon comprising inner and outer annular balloon walls spaced apart from each other and a series of inflation lumen defined between said inner and outer walls.

In a further embodiment of the present invention, said inflation lumen are defined, at least in the inflated configuration, by partition walls joining the inner and outer walls. Thin partition walls allow for a greater degree of inflation.

The partition walls of the present invention may be constructed of a material that is more resilient to stretching than a material from which the internal and/or external wall is constructed. This can help to ensure that the device of the invention inflates to a desired shape.

It is desirable that the device of the present invention has at least five partition walls, each joining the internal wall to the external wall. This arrangement ensures there are a series of inflation lumen between the inner and outer walls.

The device according to the present invention has at least one inflation lumen that narrows in a direction radially inwardly from the outer wall toward the inner wall at least in the inflated configuration. This again allows for ease of formation of the desired annular expanded configuration with a central lumen for the passage of blood and desirably each inflation lumen has such a configuration.

The device of the present invention has at least one, and desirably each, lumen that has in cross-section, a shape which is substantially that of a drop in at least the inflated configuration. Again such a shape allows for ease of achieving an annular shape in the expanded configuration with a central lumen defined therein.

The device of the present invention may have inflation lumens that are defined by a surface having folds therein. For example one or more lumen may have folds on their inner surface.

In alternate embodiments of the device of the present invention, the inflation lumens may be a substantially polygram shape in cross-section, for example a polygram selected from the group consisting of those in the range from substantially pentagram to substantially hendecagram. Such shapes can be selected based upon the desired end shape.

The device according to present invention wherein the inflation lumen are in fluid communication with each other so that they may be inflated together. This provides a system which is easy to use and possibly also greater control over the inflation and deflation of the device during deployment and re-deployment of the device in a vessel.

In an alternate embodiment of the present invention, the inflation lumens are not in fluid communication with each other and are independently inflatable. This provides great control over the inflation and deflation of the device during deployment and re-deployment in tortuous blood vessels.

The device of the present invention may further comprise at least one non-inflation lumen defined within the body. Non-inflation lumen an also assist is reducing the tendency of material to bulk together, particularly in those areas which may experience a net compressive force for example due to expansion of inflation lumens.

The device of the present invention has a central lumen diameter of at least about 1.0 mm, for example at least about 1.5mm, preferably at least about 2.0mm, such as at least about 2.5 mm, for example at least about 3.0 mm, desirably at least about 3.5 mm when in the inflated state. This ensures there is sufficient blood flow past the device. Desirably the device is connected to a delivery device/carrier such as guide wire by a mechanism which does not occlude the central lumen to any substantial extent.

In one arrangement and in the non-inflated state the body is stretch-fitted over a delivery device. Stretch-fitting ensures the device is compactly held on the device and with a low profile which aids insertion/removal.

The annular body of the device of the present invention can be tethered distally to a guide wire or alternately be tethered proximally to a guide wire or can be tethered at proximal and distal ends to a guide wire.

In a further embodiment of the present invention, the device comprises a mechanism to stretch the annular body in a substantially axial direction. Again this helps with reducing the profile for delivery/removal. Desirably also the mechanism allows contortion of the device by twisting, again to help reduce its profile.

In one embodiment, the annular body of the device of the present invention is constructed of a compliant material. This ensures compliance to vessels of different shapes. The inflation lumens of the device may comprise non-compliant balloons. For example non-compliant balloons may occupy the inflation lumen of a compliant annular body. The use of non-compliant balloons in this manner can ensure that inflation always achieves the same degree of expansion of the device.

In a still further embodiment of the present invention, an annular sheath of elastic material with a series of lumen defined therein provides the annular body. This thus provides a two-part arrangement, a sheath and balloons which occupy lumens in the sheath. This allows flexibility. For example the same sheath may be mated with non-compliant or compliant balloons as desired. It will be appreciated that a compliant or non-compliant sheath may be used. A compliant sheath may be preferable because its resilience will effect bias toward the non-expanded state when inflationary pressure is removed. If there is no such resilient bias in the device, which may occur for example where a non-compliant sheath is used then returning the device to the non-expanded configuration can be achieved utilising suitable means, for example by vacuum. Optionally the lumen in the annular body each house a non-compliant balloon.

The device of the invention optionally further comprises filter and/or infusion functionality.

For example a further embodiment of the device of the present invention includes a filter which extends across the internal lumen and which can act as an intraluminal embolic capture device to capture embolic material from blood passing through the internal lumen. The filter will open out and collapse with the device.

Optionally the filter is attached to a distal end of the annular body or is attached to a proximal end of the annular body.

It is desirable that the annular body extends over a substantial portion of the length of the filter. This means that the inflation and deflation of a device of the invention will ensure opening out and collapsing of the filter. In particular this may ensure the mouth of a filter is occluded in the non-inflated configuration to ensure material caught by the filter does not escape as the device of the invention is removed following deployment.

In a still further embodiment of the device of the present invention, the device comprises an infusion means for infusing a substance into a vessel. It is desirable to be in a position to release a therapeutic agent into a vessel to assist in removal of occlusions etc.

The device may be adapted to infuse the substance when a predetermined inflated pressure in the annular body is reached. Automatically then when a pressure is reached the therapeutic material may be released. For example the device may comprise one or more rupturable reservoirs which house the substance to be released and wherein the reservoirs are ruptured in use to release the substance. Rupture may occur when a certain pressure is reached.

Alternatively or additionally the device of the invention may comprise infusion tubes on the annular body. The infusion tubes may be held to the annular body by elastic cuffs. Alternatively they may be directly bonded thereto, for example utilising adhesive. The tubes will be flexible so as to not impede the movement of the device from its non-inflated to inflated configurations and vice versa.

In an alternate embodiment, the device further comprises a perforated sheath of elastic material enveloping the annular body which allows release of the material. Alternatively the device further comprises a porous sheath enveloping the circumference of the annular body which allows for infusion. Again in such arrangements release can be triggered automatically when a certain inflationary pressure is reached or fluid can be supplied from a remote supply under user control.

In such infusion embodiments any sheath is bonded to a proximal and distal circumference of the annular body.

The present invention thus may provide a multi-lumen elastic filter scaffold. This allows the entire filter scaffold to expand and contract. The use of non-compliant balloons in the multi-lumen elastic filter scaffold is a further aspect of the invention. The present invention can also provide an all-elastic device filter scaffold which is deployable by inflation. A filter, for example in the form of a mesh may be attached, for example distally, to the multi-lumen scaffold. In addition a proximal elastic region that incorporates orifices that facilitate blood flow may be provided.

A device of the invention will open out and achieve vessel wall apposition. This means that debris cannot pass between the vessel wall and the device of the invention. The device of the invention can be adapted to infuse a solution into a vessel, for example a therapeutic solution. It will be appreciated that a device of the invention can be used to treat target sites other than occluded sites. For example a device of the invention can be employed to treat an aneurism such as a cerebral aneurism. Desirably in such an application the device of the invention is adapted to infuse a therapeutic material such as a clotting agent to the target site.

The device of the invention may incorporate a distal elastic filter mesh. This is particularly desirable for applications where the treatment may result in the creation of debris.

Axial tensioning and/or twist of the annular device can be employed to reduce the profile of the device for delivery and retrieval.

It will be appreciated that the device of the invention expands by net radially outward expansion. This contrasts to expandable devices of the prior art where a balloon expands by relative movement of the walls away from each other. Such structures are not suitable for creating an annular arrangement upon expansion which will have a central lumen defined therein which will allow sufficient blood flow through the lumen and thus the blood vessel. In contrast with the present invention the central annular lumen is enlarged sufficiently to allow fluid to flow therethrough. The device of the present invention thus forms an open-ended inflated annular structure. The central annular lumen is formed when the inner wall moves radially outwards by the inflation of the annular balloon. The area between the inner and outer walls of the expandable may form an inflatable cavity which may be partitioned into inflatable lumens which in turn can be independently inflated or arranged in communication so as to be inflatable together. Upon expansion the annular body opens out to form the annular structure of the present invention.

In the present invention the expandable annular body may be formed by a compliant or non-compliant material such as by a compliant balloon or a non-compliant balloon. If a noncompliant balloon is employed, it is desirable to use it in combination with a multi-lumen elastic component.

For certain applications the diameter of the internal lumen may be relatively large. For example the lumen may have a diameter in the range from about 4 mm to about 12 mm. Such a diameter may be useful for peripheral applications. For other applications the lumen may have a diameter from about 1 mm to about 4 mm.

It will be appreciated that the expandable annular body of the present invention will be biased by expansive forces towards an expanded configuration which is desirably substantially cylindrical in shape. However, it will be appreciated that the expandable annular body will take up the shape of a vessel in which it is placed and against which it abuts in the expanded state. For example in the expanded state of the expandable annular body may take up, upon expansion, a substantially elliptical shape of a vessel in which it is placed.

Lumen defined within the annular body can be of any desired shape. For example they may be star-shaped in cross-section.

Stretch fitting of the device onto a carrier is desirable. Stretching can help to reduce the profile of the device further. For example, axial stretching can elongate the device and reduce its (height) profile. Stretch fitting may be applied up to 50% of the maximum strain of the device. Where a filter devices is employed, stretch-fitting ensures that the filter device collapses upon removal of the expansionary force on the annular body. This helps to trap debris and ensure it is not lost during removal of the device.

Desirably the expandable device of the invention is constructed of elastic material. Suitable materials include an elastic polyurethane or silicone. The filter may also be constructed of the same material. For example a polyurethane mesh may be used to form a filter. Desirably the filter takes the form of a basket.

The device of the present invention can be mounted on a carrier such as a guide wire so for example that one end is free to move axially along the guide wire when pulled, while the opposite end remains fixed to the guide wire, to reduce the profile of the annular balloon. A tether may be used in which case the tether may comprise a fluid delivery tube for delivery of expansionary fluid to the annular body.

The present invention extends to an expandable annular device substantially as described herein and/or as illustrated in the accompanying drawings.

### Brief Description of the Drawings

The invention will be more clearly understood from the following description of an embodiment thereof, given by way of example only, with reference to the accompanying drawings, in which:-

**Figures 1A and 1B** illustrate a perspective and front elevational view, respectively, of one embodiment of an annular device for use in the present invention in an inflated configuration; **Figure 1C** shows (a segment of) sequence of cross-sectional views of the device of Figure 1 in various states of expansion starting with a non-inflated state Fig 1 C(i), to a fully expanded state in Fig 1C(v);

**Figures 2A, 2B, 2C, 2D, 2E and 2F** provide cross-sectional views of illustrative shape variants of the inflation lumen of a multi-lumen annular device for use in the invention;

**Figures 3A and 3B** illustrate a perspective and a front elevational view, respectively, of an annular device for use in the invention comprising interconnected inflation lumens; **Figure 3C** illustrates a side-sectional view of the device of Figure 3A while **Figures 3D - 3F** show respective cross-sectional views of the device of Figure 3 along lines A-A to C-C respectively;

**Figure 4A** illustrates a perspective view of an annular device for use in the invention comprising independently inflatable inflation lumens; **Figure 4B** shows a perspective view of an annular device Figure 4A with the inflation lumens shown in dashed outline; **Figure 4C** illustrates a front elevational view of the device of Figure 4A; **Figure 4D** shows a side elevational view of the device of Figure 4A **Figures 4E - 4G** show respective cross-sectional views of the device of Figure 4A along lines A-A to C-C respectively;

**Figures 5A and 5B** illustrate a side view of a device for use in the present invention mounted on a carrier and including a mechanism to reduce the device profile, the mechanism being in a non-active state in Figure 5A and the mechanism being in an active state in Figure 5B resulting in a reduced device profile;

**Figures 6A, 6B and 6C** show a perspective view, front elevational view, and side elevational view respectively, of a device for use in the invention with a filter attachment;

**Figure 7** illustrates a side elevational view of a device for use in the invention with a filter attachment wherein the filter attachment is housed substantially within the lumen of the device;

**Figures 8A and 8B** provide perspective and front elevational views, respectively, of a device of the invention provided with a sheath and tethered to a guidewire; **Figures 8C** provides a perspective view of the device of Figure 8A with the sheath removed while **Figure 8D** provides a perspective view of the sheath;

**Figures 9A and 9B** provide perspective and front elevational views, respectively, of a device of the invention provided with a sheath and tethered to a guidewire; **Figures 9C** provides a perspective view of the device of Figure 9A with the sheath removed while **Figure 9D** provides a perspective view of the sheath;

**Figure 10A** is a cross-sectional view of a device for use in the invention adapted to infuse therapeutic materials, in position within a vessel; while **Figure 10B** shows an enlarged view of Detail A of Figure 10A, **Figure 10C** shows a schematic representation of a rupturable reservoir suitable for use with the embodiment of Figure 10A and 10B;

**Figures 11A and 11B** illustrate cross-sectional views of a device for use in the invention which is adapted for infusion, in a normal and occluded vessel, respectively.

**Figure 12A** illustrates a side elevational view of a device for use in the invention which is adapted for infusion and including a cuff comprising infusion tubing; **Figure 12B** is a perspective view of the infusion cuff of Figure 12A;

**Figure 13A** illustrates a side elevational view of a device for use in the invention which is adapted for infusion and including a cuff comprising infusion tubing and a perforated sheath while **Figure 13B** illustrates a side elevational view of a device for use in the invention which is adapted for infusion and including a cuff comprising infusion tubing and a sheath constructed of porous material;

**Figures 14A and 14B** illustrate cross-sectional views of a device of the present invention in a circular and oval vessel, respectively;

**Figures 15A and 15B** are photographs of a device constructed in accordance with the present invention in a circular and oval vessel, respectively; and

**Figures 16A and 16B** respectively show a transverse sectional view and a longitudinal sectional view of a device of the invention in use to treat an aneurism in a vessel.

### Detailed Description of the Drawings

Various embodiments of the present invention will now be described with reference to the accompanying Figures. All statements about features, materials or parameters can be applied to all embodiments of the invention.

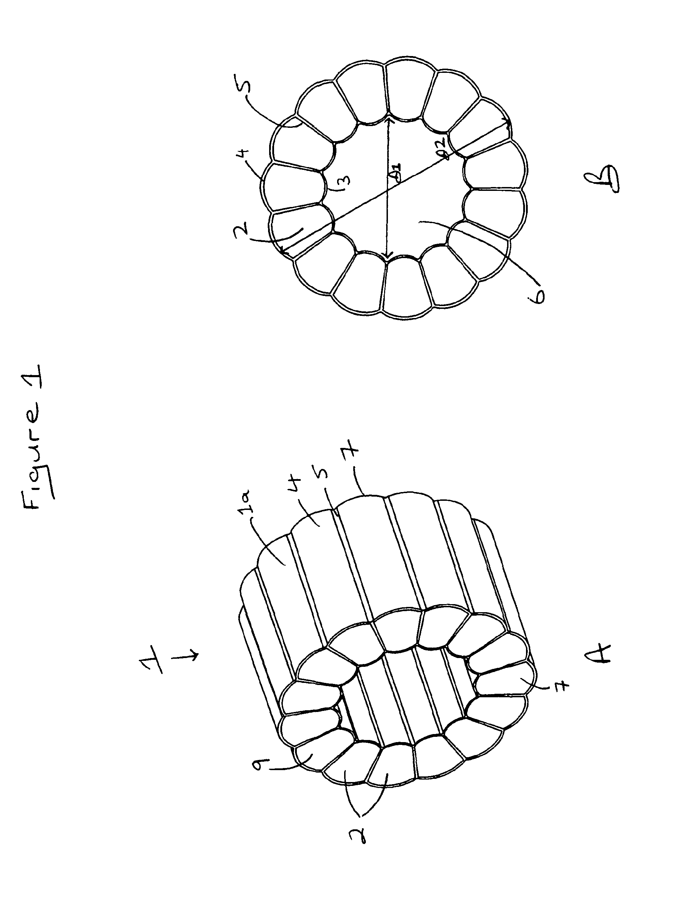

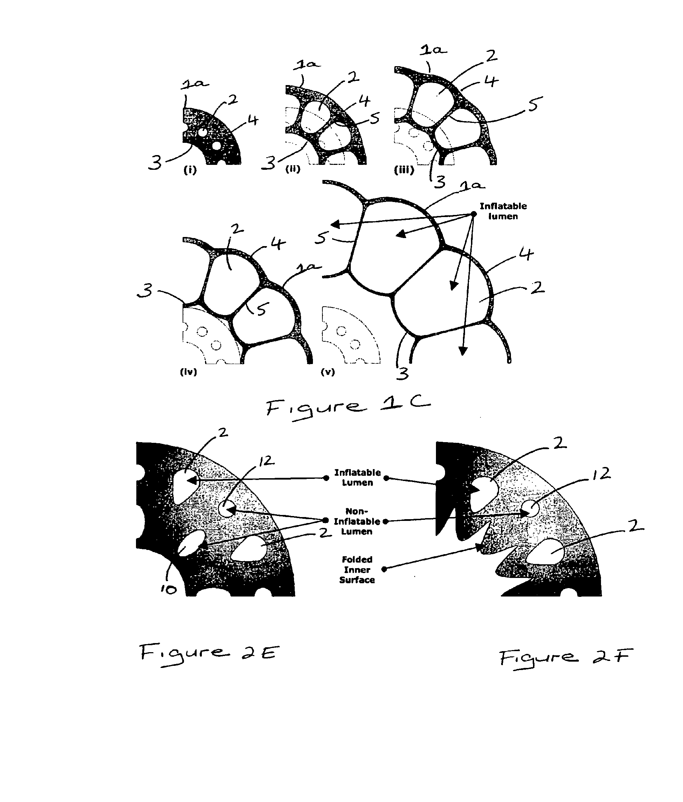

In Figure 1 there is illustrated a perspective view and front view of a device for use in the present invention. The device is for use within the vasculature of the body. The device is generally indicated by the reference numeral 1, and is shown in an inflated state. In the embodiment shown, the inflatable device 1 comprises an annular body 1a in the form of an annular balloon. The device is expandable by a series of inflation lumens 2 defined in the annular body 1 a. The device has inflated and non-inflated states. Upon expansion of the expandable body the device moves from a non-inflated to an inflated (expanded) configuration. In Figure 1 it is shown in its inflated state. As with all embodiments of the present invention the device is biased towards its non-inflated configuration so that removal of the expansion force will cause the device to move from an inflated configuration toward a non-inflated configuration.

The device 1 further comprises an inner annular wall 3 and an outer annular wall 4. Together these walls define an annular structure, which is typically in the form of a hollow cylinder. Front and rear end walls 7,8 (running transversely to the inner and outer walls 3,4) close the annular structure. An inflatable cavity 9 is formed between inner and outer walls 3,4. In the embodiment, the cavity 9 is partitioned to define inflatable lumen. In particular partition walls 5 run (radially) between the inner and outer walls and substantially axially along the device 1 so as to form longitudinal lumens 2 within the annular body 1a. In the embodiment the longitudinal lumens 2 are inflatable. A central lumen 6 allows for blood flow through the device. When in position within the vasculature the outer wall 3 abuts and forms a barrier with the vessel wall thus cutting off blood flow save through the lumen 6.

In this embodiment the inner and outer walls 3,4 are each formed by a thin membrane of material. It will be appreciated that the partition walls 5 are also constructed of a thin membrane of material. The material may be thin even in the non-inflated state, and according to one desired aspect of the invention the material thins as the device expands. In this respect the volume of the annular body has a greater cavity volume than material volume.

In certain embodiments it may be desirable that the partition walls are created from a material that is more resilient to stretching than material from which the internal and/or external wall is constructed. Selection of materials in this way can help to adjust the inflation profile of the device.

It is desirable that there are at least five partition walls each joining an internal wall to the external wall. This allows for more even distribution of the stretching forces and thus even expansion of the device.

The annular body is formed by side-by-side and circumferentially arranged lumen 2. It will be appreciated that as the device 1 expands an internal diameter D1 increases. So too does an external diameter D2.

It will be appreciated that the length of the diameter D1 or D2 can be varied depending on the degree to which the device is expanded. In all embodiments, it is desirable that the device is sufficiently expanded so that the internal lumen 6 (which will have the same diameter as D1) allows sufficient blood flow through the device.

The inflation lumens 2 narrow in a direction radially inwardly from the outer wall 4 to the inner wall 3. Each thus forms a circumferential segment of the annular device. In the inflated state, the annular body expands radially outward to grip a vessel wall and enlarges sufficiently to allow blood flow through the central lumen 6. The shape of the inflation lumens 2 as depicted in Figure 1 is substantially that of a drop. Such a shape allows for desirably expansion of the device.

One of the issues then arises with inflating devices, for example with fluid pressure, it is to achieve the desired end configuration of the device. Some constructions of prior art devices include balloons which are mounted on a guide wire. Typically, such mounting of the balloons means there is no central lumen through which bodily fluids such as blood can pass. Often, in such cases, the balloon is mounted on a rigid carrier, such as a rigid conduit. In such cases one wall (an inner side) of the balloon may be fixed in position and it is only the other (outer side) wall that can expand in response to expansionary pressure. In such a case only the external diameter of the balloon tends to increase when inflation occurs. In other devices the balloon does not have an annular shape, instead it is provided with a neck through which inflationary fluid is provided. In such a case the opposing balloon walls expand away from each other to a substantially equal extent.

**Figure 1C** shows in sequence (expansion in the sequence from (i) to (v)) the type of expansion which is achieved with a device of the present invention. For convenience of illustration only a segment of the device is shown in this sequence. Figure 1C (i) shows the original configuration of the unexpanded device while Figures 1C (ii) - (v) show the original configuration of Figure 1C (i) in ghost outline (for comparative purposes), while the expanded configuration is shown in full outline.

**Figure 1C****(i)** shows the annular body 1 a in an unexpanded configuration. (It will be appreciated that the profile of the annular body 1 a may be further reduced for delivery/travel within the vasculature as will be described in more detail below). Figure 1C(i) shows the annular body 1a before any expansion force is applied. **Figure 1C** **(ii)** shows the effect of expansionary forces on the device. In particular each of the lumen 2 is being inflated by an inflationary fluid. As can be clearly seen by comparison with the device as shown in Figure C (i) the inflationary pressure causes outward radial expansion of both the inner and outer walls 3,4. **Figure 1C****(iii)** shows the effect of continued expansion as does **Figure 1C****(iv)** and **Figure 1C****(v)**. It will be appreciated that upon removal of the expansionary force the sequence will be reversed as the device contracts.

While there are a number of effects upon inflation, the desired overall effect for all embodiments of the invention and which is clearly demonstrated in the Figures is that there is substantial net expansion of the cylindrical ring of the annular body radially outwardly. Figure 1C(v), in particular highlights the substantial outward net radial movement of the entire annular body that has occurred from the non-inflated to the inflated states. (The configuration of Figure 1 C(v) is the same as that shown in Figure 1A and 1B). The position of the inflation lumens is critical to this process and accordingly the arrangement of the lumens within the annular body will be selected to cause substantial outward net radial movement of the entire annular body upon inflation of the inflation lumens.

As is desirable in all embodiments the inner diameter (D1) of the device in the inflated state is substantially greater than the outer diameter (D2) of the device in the non-inflated state. Typically the outer diameter will be in the range from about 1 mm to about 12 mm, preferably about 4 mm. Typically the inner diameter will be in the range from about 0.6 mm to about 10 mm, preferably about 3 mm. Typically the device when expanded has an outer circumference of the order of from about 3 to about 10 times that when in an uninflated state. Typically, when expanded, the cross-sectional area of the device occupies no greater than about 50%, suitably no greater than about 40% of the cross-sectional area of a blood vessel in which it is placed. When moving from the uninflated to inflated states it is desirable that the degree of expansion that occurs is such that the outer circumference of the device increases at least about three-fold in size, for example, at least about four-fold.

The device of the invention may be composed of compliant or non-compliant material. Non-compliant material may be utilised to ensure that the device has set inflated dimensions. Compliant material may be used where compliance of the device may be desirable to ensure that it can expand to a variable extent by stretching so that the device of the invention may be used in different sized and/or shaped vessels. Desirably the annular body 1 a of the expandable device 1 in the present embodiment is composed of a compliant material.

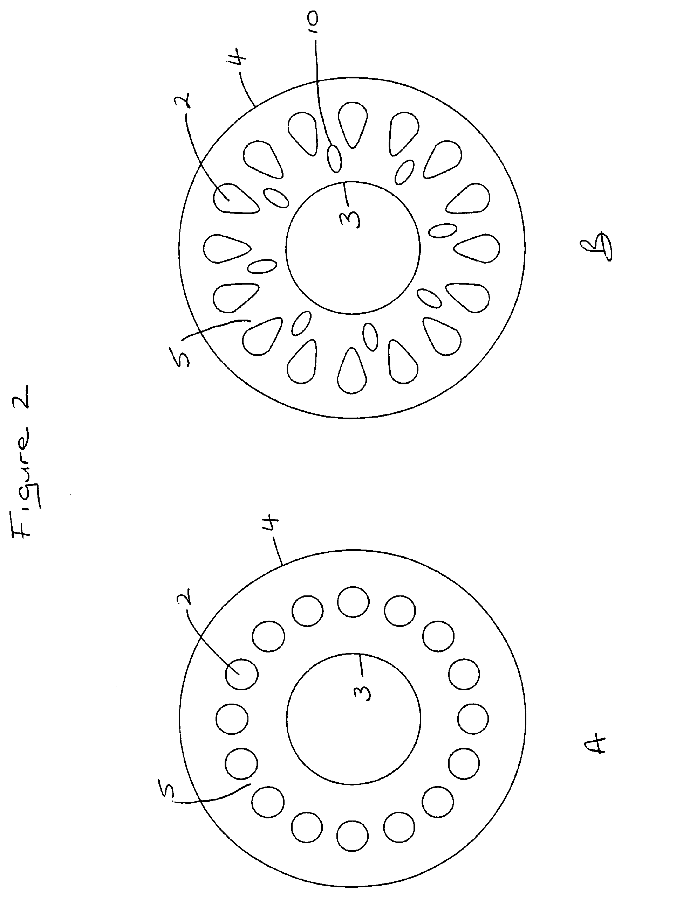

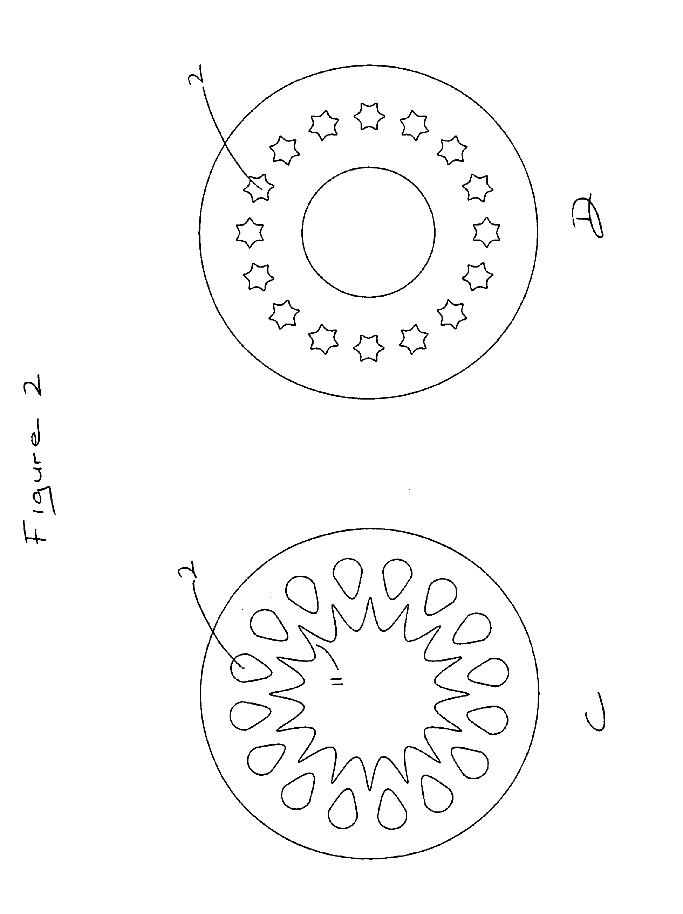

With reference to Figure 2, where the inflation device is in a deflated state, it can be seen that a variety of shapes may be adopted for the inflation lumens 2 of the inflatable device 1. **Figure 2A,** is a cross-sectional views of a device 1 which is similar to that in Figure 1. For example, the inflation lumens 2 are depicted as being circular in shape in Figure 2A, a teardrop shape in **Figures 2B and 2C,** and the inflation lumen have a shape having folds or rebates 11 therein such as in the substantially polygram shaped lumens, star-shaped lumens in **Figure 2D.** As the portion of the annular balloon that experiences most stretch is the material lining the surface of the inflation lumens 2, the lumen shape is not a significant factor on the ultimate shape of the expanded device. The inflation pressure expands the inflation lumens 2 in a radial manner so that the expanded inflation lumen 2 tends toward a circle regardless of the shape of the inflation lumen 2. Desirably the non-inflation lumens 10 are evenly distributed around the annular body. It is also desirable that as shown in Figure 2B, the non-inflation lumens 10 are disposed radially inwardly of the inflation lumens 2.

In a further embodiment of the present invention, the inflatable device 1 further comprises a series of non-inflation lumens 10 (Figure 2B) and/or folds/rebates 11 (Figure 2C) on the inner surface of annular body 1 a. The non-inflation lumen 10 and folds /rebates 11 are effective to reduce the amount of material in the annular body thus allowing for better expansion. There is less material to be compressed between the inflation lumen and there is therefore less bulging upon inflation. Such arrangements also help to ensure that the inner diameter is pulled outward with the outer diameter during expansion. For example the folds or rebates 10 are effective in reducing the material about the internal diameter, which also ensures the internal diameter is pulled outward with the outer diameter during expansion.

**Figure 2E** shows a further embodiment where there are a series of inflatable lumens 2 and two series of non-inflation lumens. In particular there is a first series of non-inflation lumens 10 (which correspond generally to those shown in Figure 2B). A second series of non-inflation lumens 12 are also shown in Figure 2E. These non-inflation lumens 12 are defined within the annular body at a position radially outwardly from the first series of non-inflation lumens 10.

**Figure 2F** shows an embodiment with folds/rebates 11 similar to those of the embodiment of Figure 2C but additionally having non-inflation lumen 12.

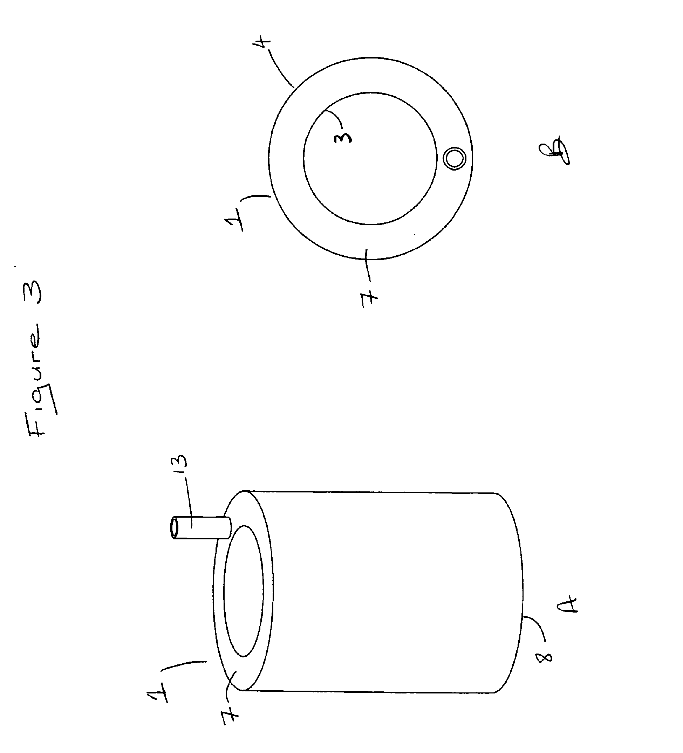

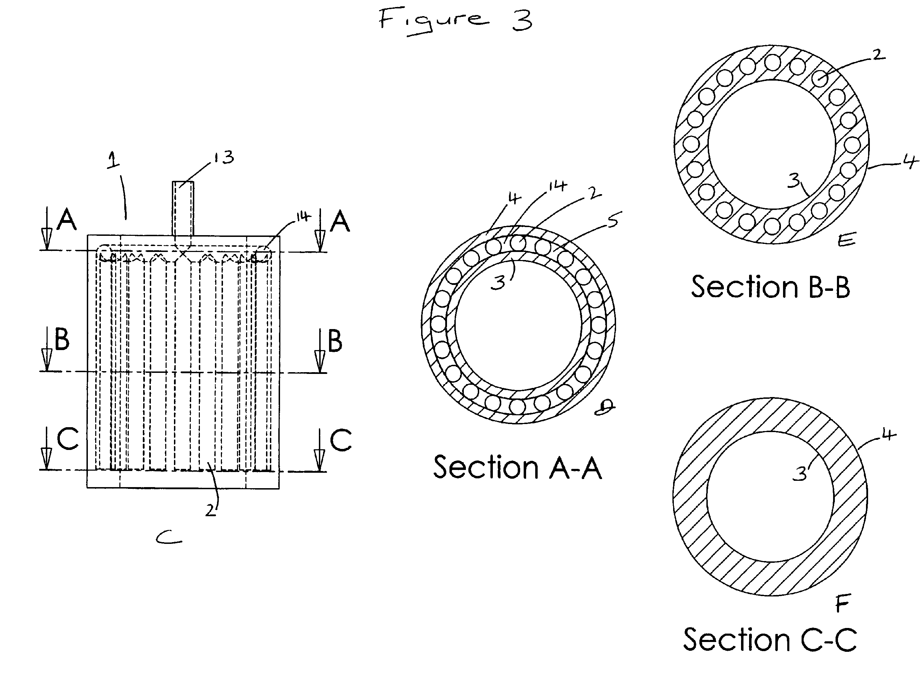

Referring now to **Figures 3A and 3B,** there is shown an inflatable device 1 wherein the inflation lumens 2 are in fluid communication with each other so that they may be inflated together. The inflation lumens 2 are inflated by introducing fluid pressure via an inflation port 13. In the embodiment the lumens 2 do not occupy the entire length of the device so that profiles of the lumens 2 are not visible at the front wall 7 or rear wall 8.

**Figures 3C to 3F** show the internal structure of the device 1. A connection conduit 14 is in fluid connection with each of the lumen 2 and the port 13 (as best seen from Figure 3C and 3D). The connection conduit 14 is in the form of a ring which runs about one end of the annular body. The inflation lumens 2 are connected together so that the inflation lumens 2 are inflatable together.

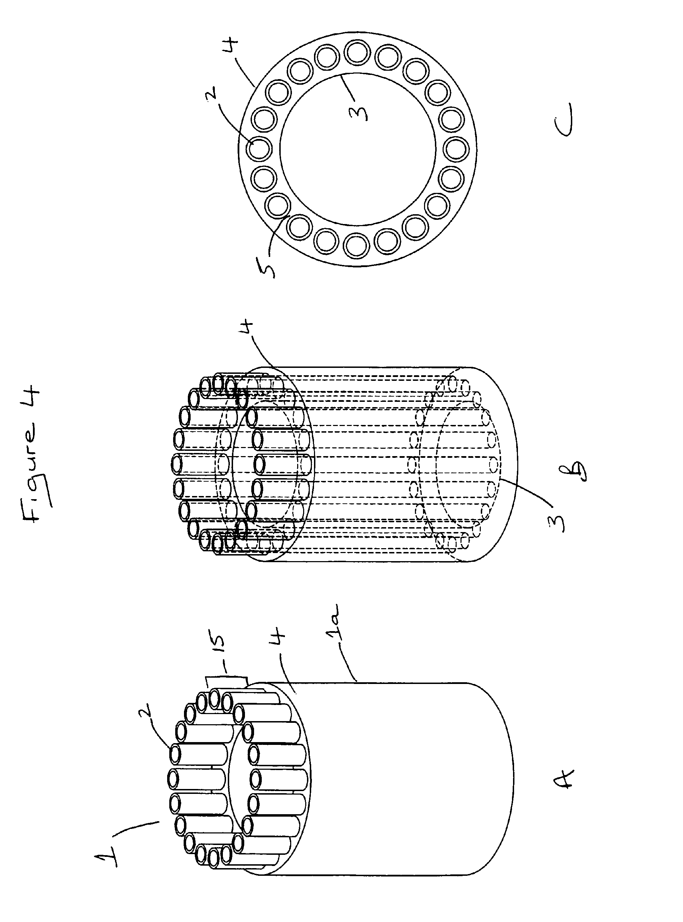

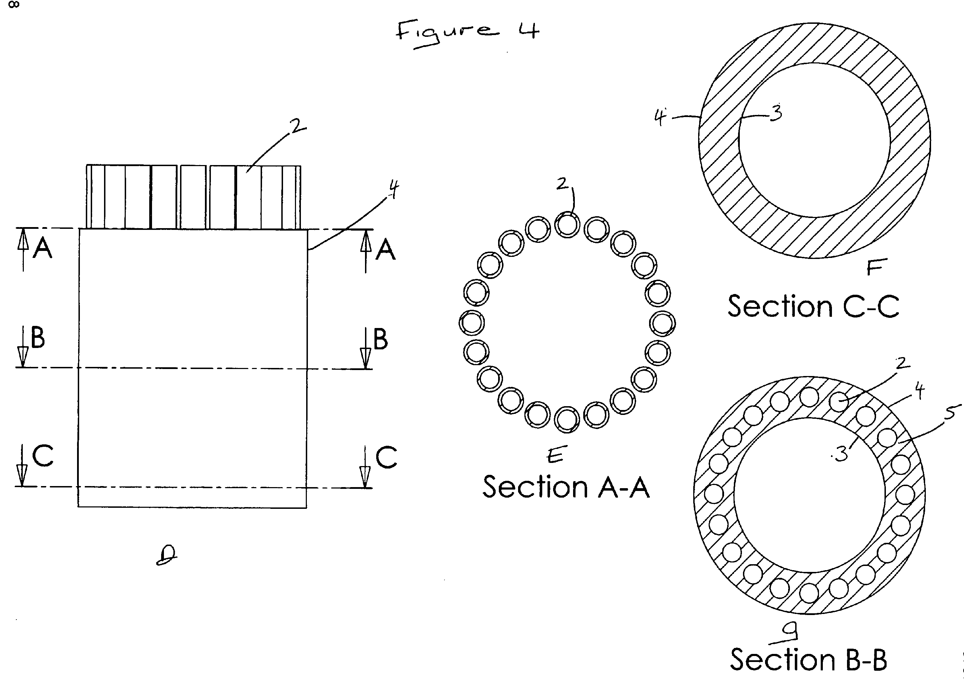

With reference to **Figures 4A-4G**, there is shown an alternate embodiment where the inflation lumens 2 are not in fluid communication with each other so that the inflation lumens 2 can be inflated separately. Each lumen 2 has a lumen defining portion 15 which projects beyond the annular body 1 a. The lumens are not interconnected so that they can be independently inflated. Independent inflation can allow more control of how expansion occurs and the shape of the device as it expands.

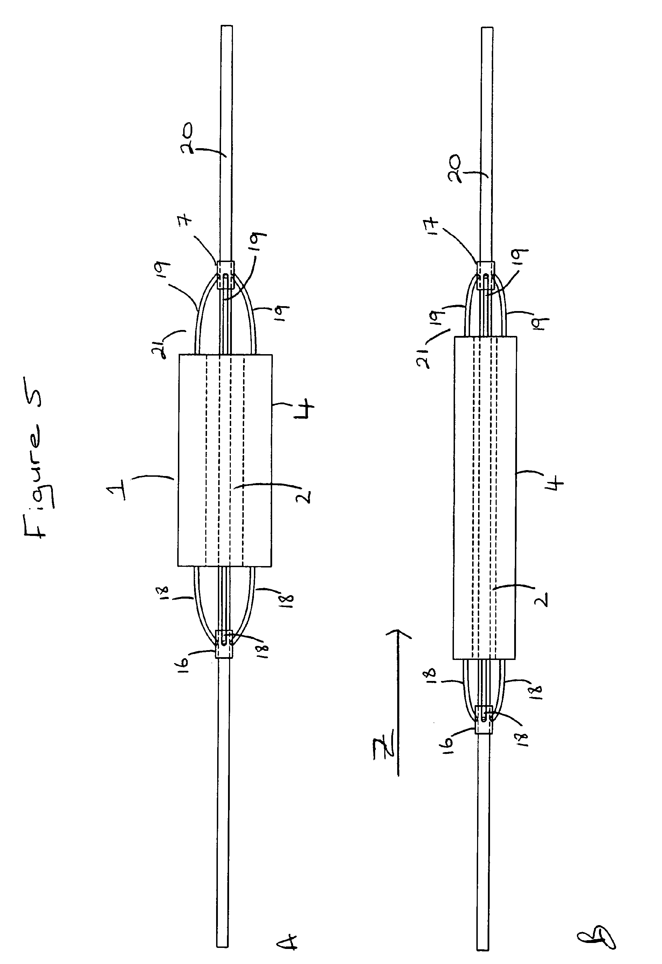

It will be appreciated that each device of the invention has an inflated, and a non-inflated configuration. Each device of the invention will also have a "rest" or "natural" configuration when it is uninflated. In other words the device will tend to a particular shape and size when it is not under any inflationary pressure. That shape and size will be smaller than that which is manifested upon exertion of inflationary pressure. It may be desirable nonetheless, to reduce the profile of the device for particular application for example insertion into the vasculature. Accordingly, it may be desirable to reduce the profile of the device to a profile which is smaller that that of the natural uninflated configuration. A configuration of the device suitable for such purposes is shown in **Figures 5A and 5B.**

Figures 5A and 5B show a device 1 of the invention mounted on a carrier which in the embodiment is a guide wire 20. A mechanism 21 is provided for reducing the profile of the device 1. In particular the mechanism allows a user to pull and/or twist the inflatable device 1 prior to and/or post deployment, in order to reduce the radial profile of the inflatable device 1. A proximal cuff 16 is attached to a first end of the inflatable device 1 via flexible connecting arms in the form of hollow tubing 18, while a distal cuff 17 is similarly attached thereto (at a second end) via flexible connecting arms in the form of hollow tubing 19. Inflation lumens 2 are shown in dashed outline.

Both the proximal cuff 16 and distal cuff 17 are mounted on a guide wire 20. In the present embodiment, the proximal cuff 18 is fixed to the guide wire 20, while the distal cuff 17 is slidably attached to the guide wire 20. To axially stretch the inflatable device 1 (from its rest position as shown in Figure 5A) it is pulled proximally in the direction of the arrow Z utilising the proximal cuff 16. The device is thus stretched axially and the radial profile of the inflatable device 1 is lowered (as shown in Figure 5B). It will be understood by those skilled in the art that the proximal cuff 16 may be slidably attached to the guide wire 20, while the distal cuff 17 may be fixed to the guide wire 20 so that pulling the distal cuff 17 distally may lower the radial height of the inflatable device 1. If desired both cuffs could be moveable to allow reduction of the radial profile. It will also be appreciated that release of the stretching forces will allow the device of the invention to return to its rest configuration. It will be appreciated that the mechanism of the invention can allow relative rotation of opposing ends of the device 1 to reduce the profile of the device by rotation of one end of the device relative to the other (twisting action).

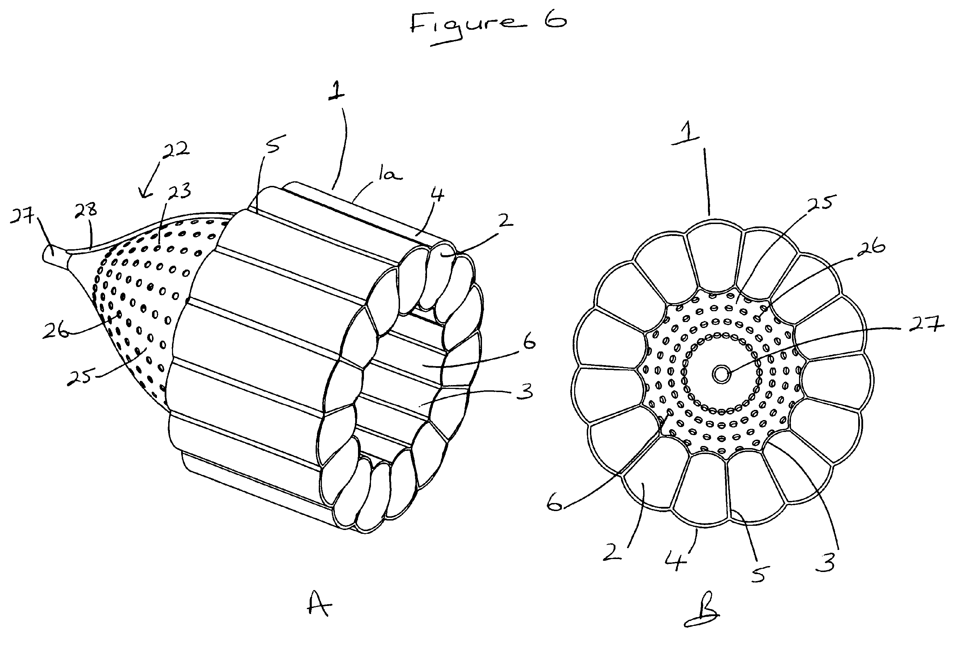

A further embodiment of the present invention is to provide a device suitable for filtration applications such as embolic filtration. **Figures 6A - Figure 6C** show such a device. Figures 6A-6C show a device 1 which is similar in construction to that shown in earlier embodiments (such as in Figure 1) with a filter attachment 22 attached to an annular body 1 a. The device is shown in an expanded configuration deployed for use. Fluid, such as blood passing through the central lumen 6 is filtered by the attachment 22.

In the embodiment distal filtration is provided by the attachment 22, which takes the form of a filter basket 23. The mouth 24 of the filter basket is joined to the annular body 1 a so that the filter opens up and out and collapses with the annular body. A filter mesh 25 comprising apertures 26 (such as micro-holes) filters fluid passing through. Debris or other undesirable particles in blood, for example debris created during removal of plaque etc from a vessel, will be caught by the filter basket 23. The filter mesh 25, which extends across the internal lumen 6 of the inflatable device 1, acts as an intraluminal embolic capture device to capture embolic material from blood passing through the internal lumen 6. The filter mesh 25 depicted in Figures 6A-6C is incorporated into a proximal end of the inflatable device 1. It may be secured to a carrier, for example a guide wire via a cuff 27. The filter mesh 25 may be constructed from a sheath of material which is the same as the material from which the annular body 1a is constructed. As such, one end of the inflatable device 1 with the filter mesh 25 is closed-off. When the annular body is collapsed to a rest state or is further reduced in profile, for example using a mechanism such as shown in Figure 5, material caught by the filter is then trapped and will not fall out as the device is collapsed for removal following its deployment. The use of a compliant balloon ensures excellent vessel wall apposition for a range of vessel cross-sections, and as such, provides the invention with a competitive advantage over the more traditional nitinol-based filters, which have difficulty conforming to irregular shaped vessel, i.e. vessel with elliptical or asymmetrical shaped cross-sections. The elasticity of the balloon material is also utilised during deflation, to collapse the filter and trap any collected debris prior to retrieving the device. By collapsing the opening to the filter first (the proximal side of the balloon) the likelihood of debris escaping the filter can be minimised during balloon deflation.

In the embodiment the inflatable device 1 is inflated and collapsed by a pressure feed tubing 28. The pressure feed tubing 28 may be connected to a distal side of the device 1 which would ensure proximal collapse before distal collapse. By collapsing the proximal side of the inflatable device 1 the likelihood of debris escaping the filter mesh 25 can be minimised during deflation of the inflatable device 1. However, it will be understood by those skilled in the art that a number of conceivable variations may exist in relation to the number of guide wire attachment points or the specifics of the pressure feed system. For example, in tortuous vessels where guide wire positioning becomes more difficult it may be beneficial to reduce the number of attachment points between the filter and the guide wire, which may aid in avoiding improper placement.

It is understood that the filter may be constructed as a simple filter basket rather than as an extension of the inflatable device.

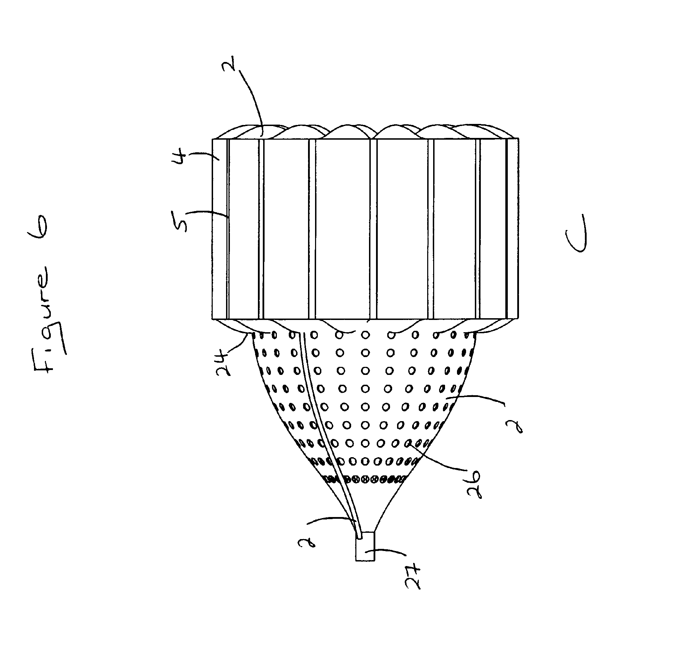

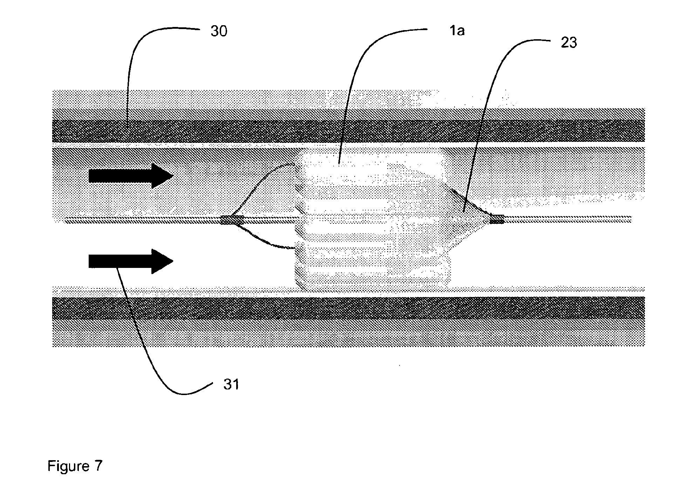

A variation of the filter devices of the invention is to house a substantial part of the filter within the annular body of the present invention. One way to achieve this is to increase the length of the multi-lumen inflatable device 1, thereby enveloping the filter basket. **Figure 7** shows such an embodiment of a device of the invention which includes a filter and which is generally similar in construction to that shown in Figure 6. The device is shown in a deployed state in a vessel 30. The arrows 31 indicate blood flow direction. In this embodiment the annular body 1a has been lengthened to accommodate a substantial portion of the filter attachment 22. The mouth 24 of the filter attachment is no longer attached to one end of the annular body 1 a but is attached internally within lumen 6 to the inner wall 3. It is desirable that at least 10% such as at least 20%, for example at least 30% in general at least 40% of the (axial) length of the filter attachment is accommodated within the annular body.

Enveloping the filter basket 23 provides greater filter stability and vessel wall apposition. The risks associated with retrieving a device using a retrieval sheath is that the filter basket may become entangled for example with a stent which is deployed during an angioplasty procedure. However, as the filter basket and trapped debris will be safely contained beneath and within the device 1 during retrieval, such risks are ameliorated.

Alternatively, the trapped debris may be removed by suction, or initially physically broken up and removed by suction.

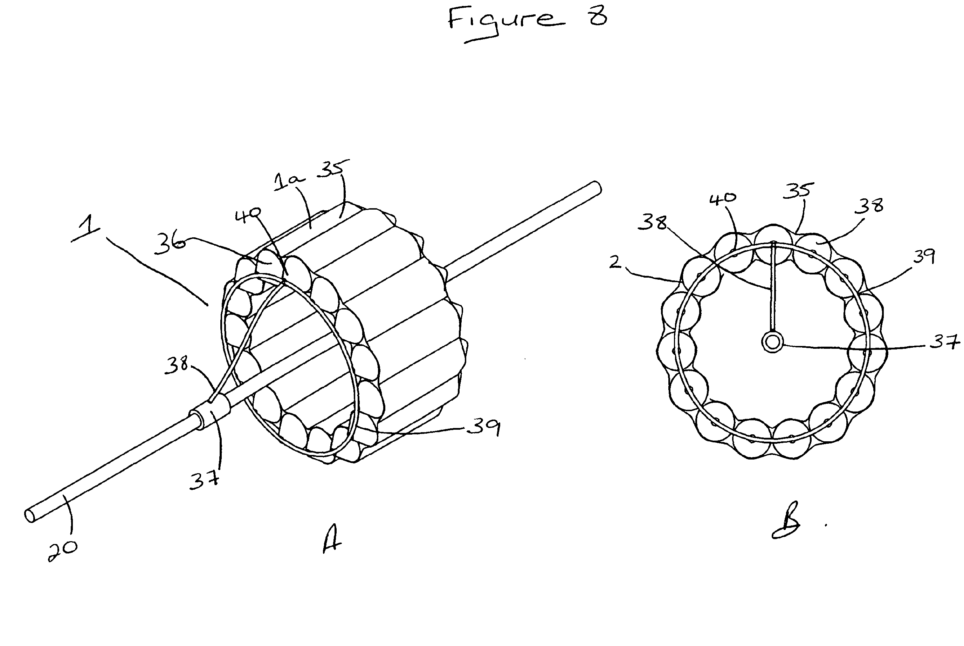

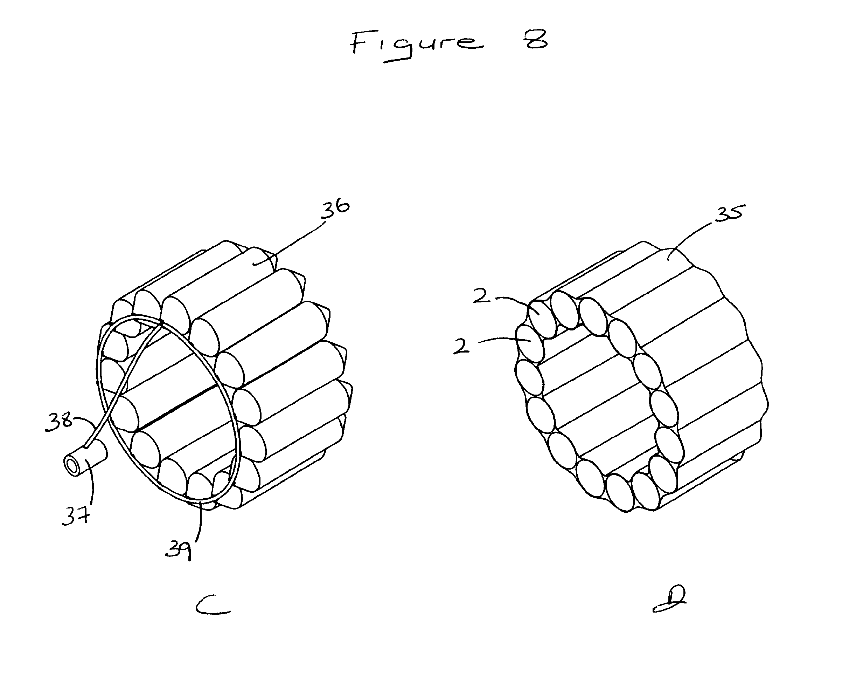

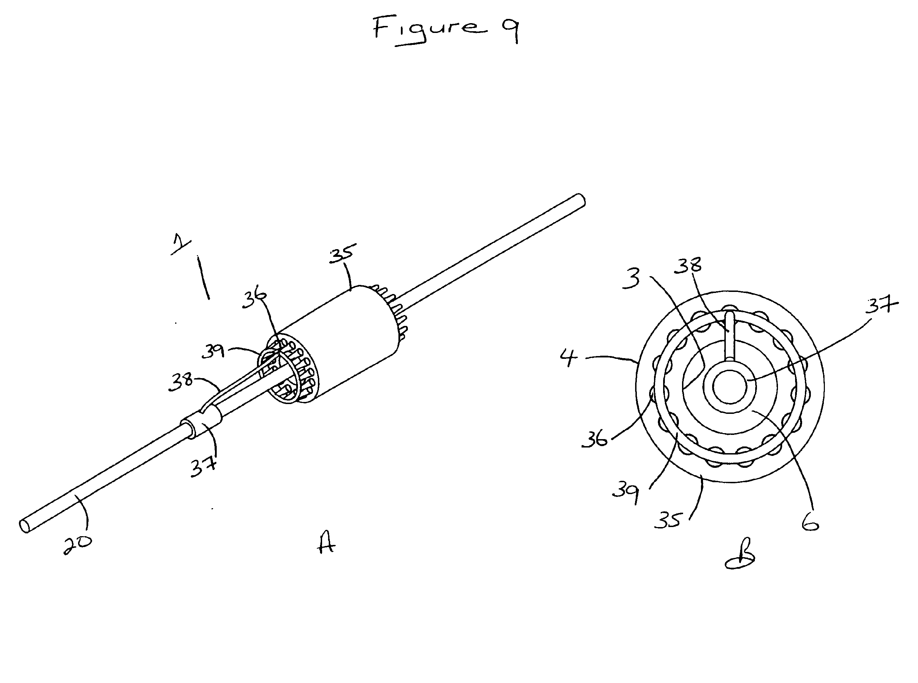

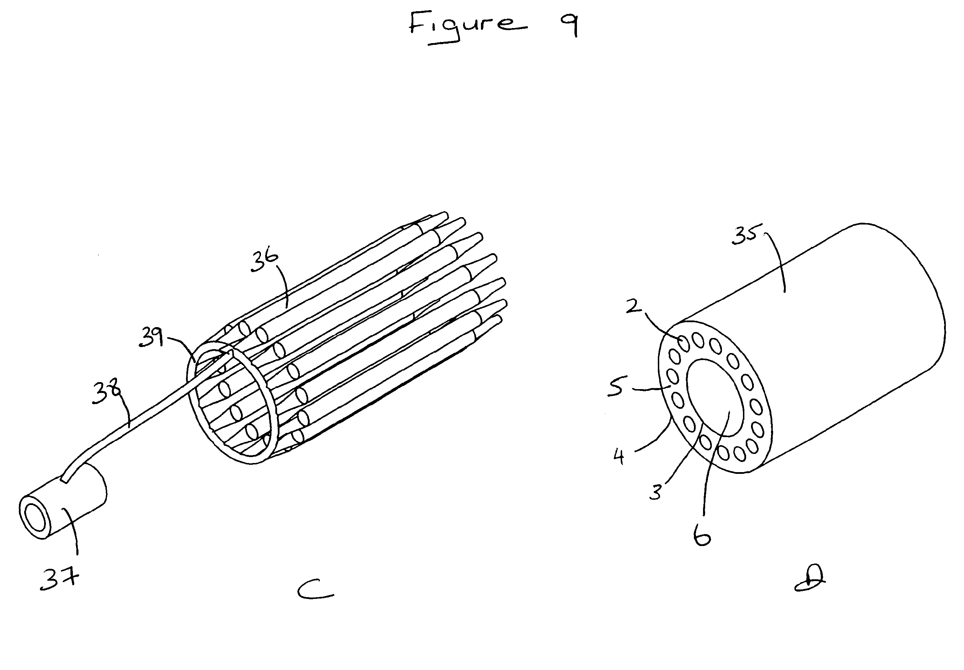

Referring now to **Figures 8A-D and 9A-D**, there is shown therein a further embodiment of the present invention wherein the inflatable device 1 comprises inflatable balloons which are insertable into an annular sheath. In this embodiment the inflatable annular body 1 a comprises two separate components, an annular sheath 35 (best seen in Figure 8D) and an annular series of balloons 36 (best seen in Figure 8C) arranged to be accommodated within lumens 2 in the sheath 35.

In the embodiment, an annular arrangement of balloons which are desirably non-compliant balloons 36 composed of non-compliant material, are inserted into the inflatable lumens 2 to form the assembly of Figures 8A and B. The expansion of the inflatable lumens 2 is initiated through inflation of the non-compliant balloons 36. Desirably the sheath 35 is elastic. It will thus function to bias the device towards an unexpanded configuration and to collapse any filter attachment as described above. When subjected to inflationary pressure it will form the desired annular structure. As with compliant balloons the non-compliant balloons can have any desired cross-sectional shape, for example they may be oval or crescent shaped. It will be appreciated that an optimal cross-sectional shape can be applied for the task in hand.

Figures 8A and 8B demonstrate the inflatable device 1 when in an inflated state. The inflatable device 1 is anchored to a guide wire 20 by a distal cuff 37. The annular body 1 a is attached to the cuff 37 by a flexible arm 38, which in the embodiment also acts as a pressure feed tube. A ring tube 39 has two functions, a first function is to act as a mount for the balloons 36 and to hold them in a suitable configuration for mating with the lumens of the sheath 35. A second function is to provide inflationary pressure for the balloons 36. A nose 40 on the end of each balloon is in fluid communication with the ring tube 39. The ring tube 39 acts as a conduit for inflationary fluid from the arm 38 to the balloons 36. The ring tube 39 and the arm 38 are flexible and allow the device to collapse.

The use of non-compliant balloons 36 within the elastic sheath 35 allows for greater control during device deployment, enabling devices to be custom produced to fit specific vessel sizes.

**Figures 9A-9D** respectively show equivalent views of each of Figures 8A-8D save that the device is in the deflated state in each of Figures 9A-9D.

It will be understood by those skilled in the art that a number of conceivable variations may exist in relation to the number of guide wire attachment points or the specifics of the pressure feed system in the above-described embodiment.

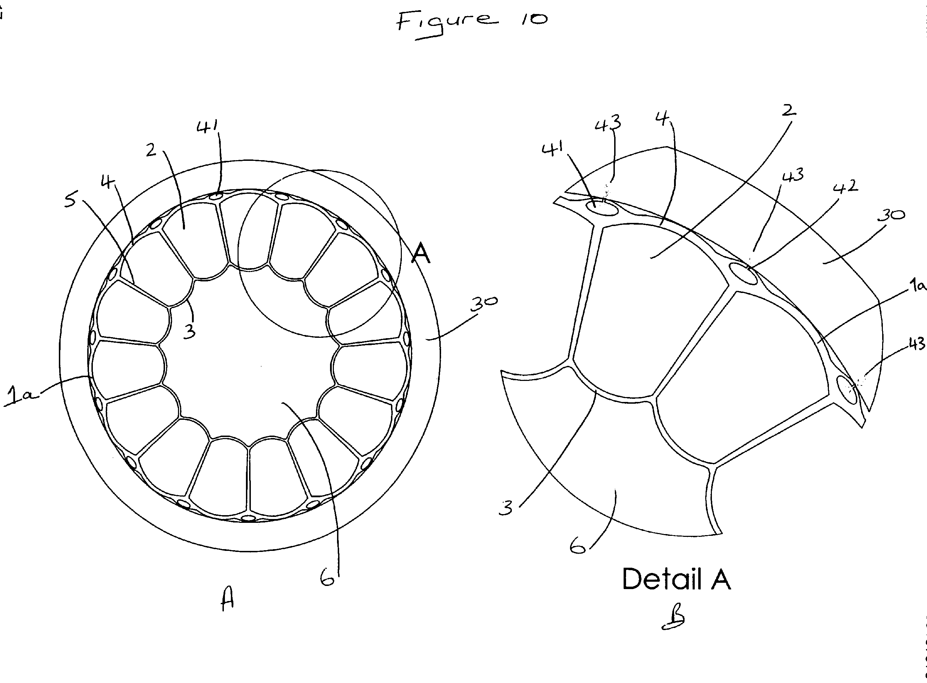

Referring now to **Figures 10A and 10B** there is shown therein a further embodiment of the present invention wherein the inflatable device 1 comprises infusion means for infusing a (therapeutic) substance into a vessel. In particular the device is adapted to infuse the material when a predetermined inflation pressure is reached.

As can be seen from the Figures the device is provided with a plurality of rupturable reservoirs which in the embodiment are a series of infusion tubes or lumen 41 disposed about the periphery of the annular body 1a. In the embodiment the device is deployed (expanded) within a vessel such as a blood vessel 30. In the embodiment a rupturable reservoir is provided on the periphery of the device and between each adjacent pair of inflation lumen 2. This allows for the even distribution of the therapeutic material to the vessel 30.

The infusion lumen 41 are provided as an integral part of the annular device. In particular they form an integral part of outer wall 4 of the device. The location of the infusion lumen 41 is chosen so as to be close to the vessel 30 following inflation. Numerous micro-holes 42 (best seen in the enlarged view of Figure 10B) in the outer surface of the outer walls 4 of the annular balloon component (coinciding with the infusion lumens 41) enables a therapeutic material 43 contained within the infusion lumens 41 to be infused into the vessel following deployment. The forced collapse of the infusion lumen 41 resulting from annular balloon component expansion combined with the compression of the annular balloon component against the vessel wall 30 ensures full evacuation of the therapeutic material 43.

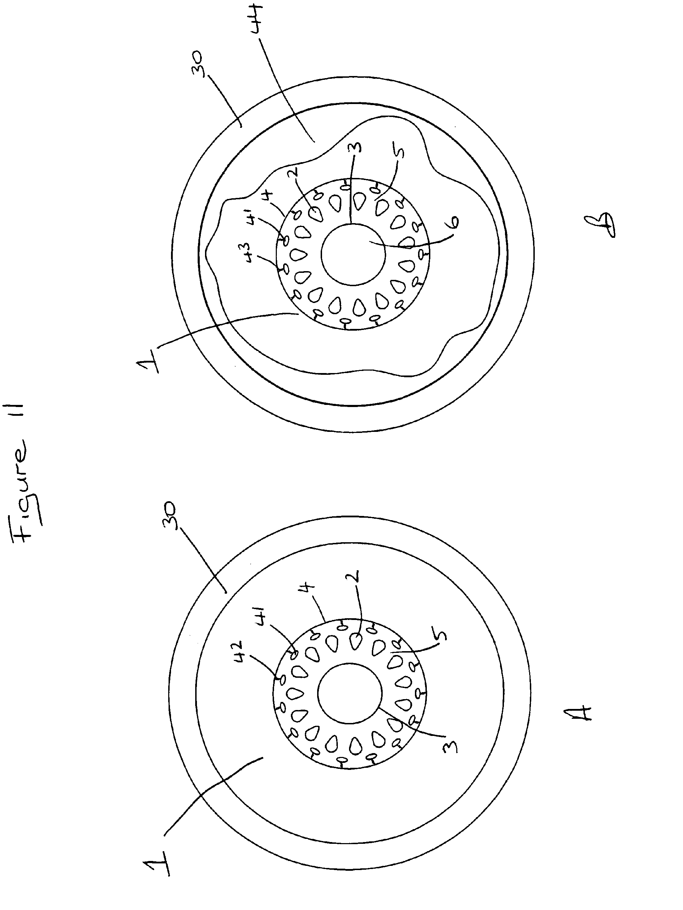

**Figures 11A and 11B** show the embodiment of Figure 10A and 10B before inflation and within the vessel 30. In Figure 11A the vessel 30 is a vessel free of plaque whereas in Figure 11B vessel 30 is shown as a diseased vessel with plaque 44 partially obstructing the vessel. When the device of the invention is a compliant one it will be able to take up the internal shape of both the non-diseased and diseased vessels.

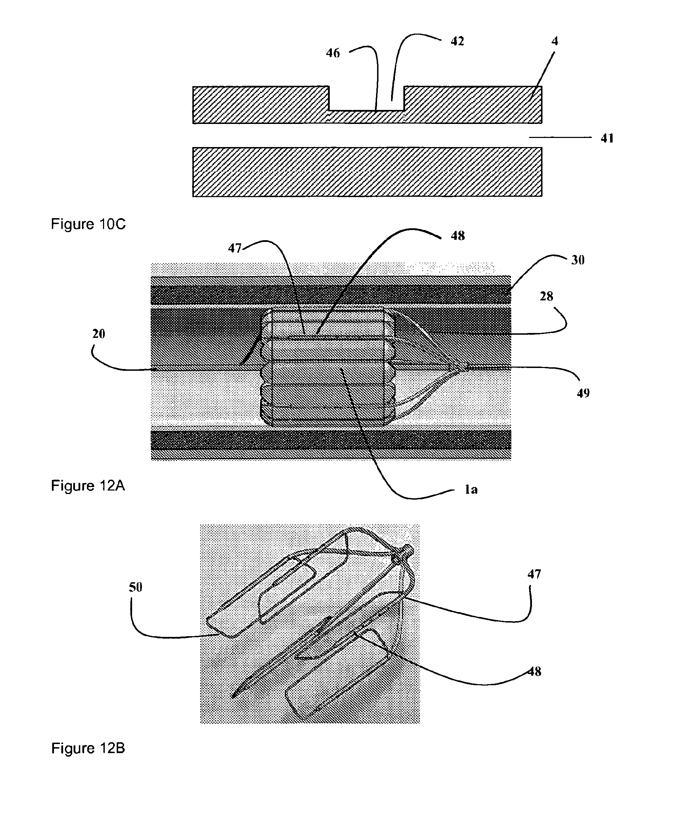

A variation of this embodiment may be to include intentional failure points as shown in the schematic representation of **Figure 10C**. In that embodiment a rupturable member 46 which functions to occlude the micro-holes 42 of the infusion lumens 41, thereby preventing the early ill-timed release of the therapeutic compound 43 and allowing for greater control over the process of drug release. The failure members may be designed to fail at a pre-determined level of expansion, so that ideally, drug release only occurs once contact has been established with the arterial wall.

A further variation of an infusion device of the invention is instead of having the infusion means as a rupturable part of the annular body, providing for delivery of the material to be infused by other means. In particular it is possible to provide one or more infusion tubes, which can deliver a therapeutic fluid. For example it is possible to house tubing within the lumen of the annular balloon component. A perceived benefit of this approach is that the therapeutic material may be injected along the length of a catheter system and as such is physician-controlled (unlike the embodiments where pressure failure is used). Assembly of the device may involve an over-moulding step to encase tubing in the annular body or alternatively, the tubing may be attached to the annular balloon component thereof.

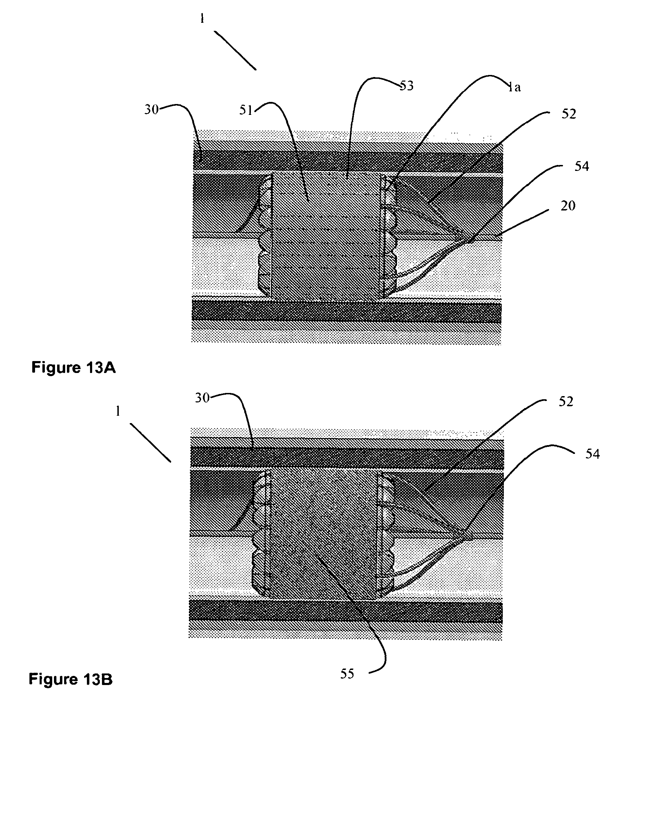

**Figures 12A, 12B****,** **13A and 13B** show embodiments of such a device. The embodiment of Figure 12 A is similar to that of Figure 5 with the annular body 1a shown in the inflated condition with a vessel 30. The annular body 1 is attached to a guide wire 20 by a series of arms 47 which form a cage which receive the annular body. The arms 47 are attached to the guide wire 20 via a cuff 49. The arms 47 function as conduits and are adapted to receive a therapeutic fluid from a therapeutic fluid source. Suitable apertures 48 in the arms 47 allow therapeutic materials to be ejected into the vessel 30 once delivered through arms 47. This embodiment allows numerous arms 47 surrounding the annular balloon component structure to be utilised. As with the previous embodiment, infusion tubing may provide fluid communication through the guide wire lumen and along the length of a catheter, thereby enabling the operator to fully control the compound delivery. The manner in which the infusion tubes are connected to the annular balloon of the inflatable device 1 must not inhibit the expansion of the annular balloon component. To ensure this, elastic cuffs 50 (best seen in Figure 12B) are used to secure the annular balloon to the arms. The elasticity of the cuffs 50 should match that of the annular balloon so that a firm connection is maintained whether the annular balloon component is in its inflated or deflated configuration.

An alternative embodiment may include the use of a sheath 51 over the annular balloon component 1a of the inflatable device 1. The sheath 51 does not impede inflation or deflation of the device and is desirably elastic. In the embodiment the sheath 51 forms a sealed cavity spanning the circumference of the inflatable device 1. (If desired, that cavity can be partitioned into separate infusion lumen) To accomplish this, the sheath 51 may be bonded to the proximal and distal circumference of the annular balloon component of the inflatable device 1 in order to create a seal. Arms 52 (which are similar to other embodiments) attach the device 1 to (a cuff 54 which in turn attaches the device to) a guide wire 20. In the embodiment the device is shown in an expanded configuration within a vessel 30.

The arms 52 form conduits for the delivery of therapeutic fluids to the cavity between the sheath 51 and the device 1 a. The therapeutic compound material thus pools within the cavity. Numerous perforations 53 on the surface of the sheath 51 allow for infusion of the therapeutic compound into the contacting vessel wall 30. A benefit of this embodiment is that the entire circumference of the vessel can be treated without the need for repositioning the device 1. Like those other embodiments that incorporate infusion tubing, the compound delivery is physician controlled.

A variation of this concept is shown in Figure 13B where there is shown a very similar arrangement to Figure 13A. In the Figure 13B embodiment there is provided a porous sheath 55. The porosity of the sheath 55 is sufficient to allow infusion of therapeutic fluid. As with the embodiment of Figure 13A, a cavity between the annular balloon component and the sheath is supplied with therapeutic fluid by arms 54. The cavity collects the therapeutic material and releases it at a desired rate by way of capillary action through the thickness of the sheath 55. Therapeutic material is thus released around the circumference of the device 1. In doing so, less of the material will be taken away by the blood stream and this increases the likelihood that successful infusion to the vessel wall will occur. The porous sheath 55 must expand along with the annular balloon component and as such, a suitable material such as an elastomeric foam may be required.

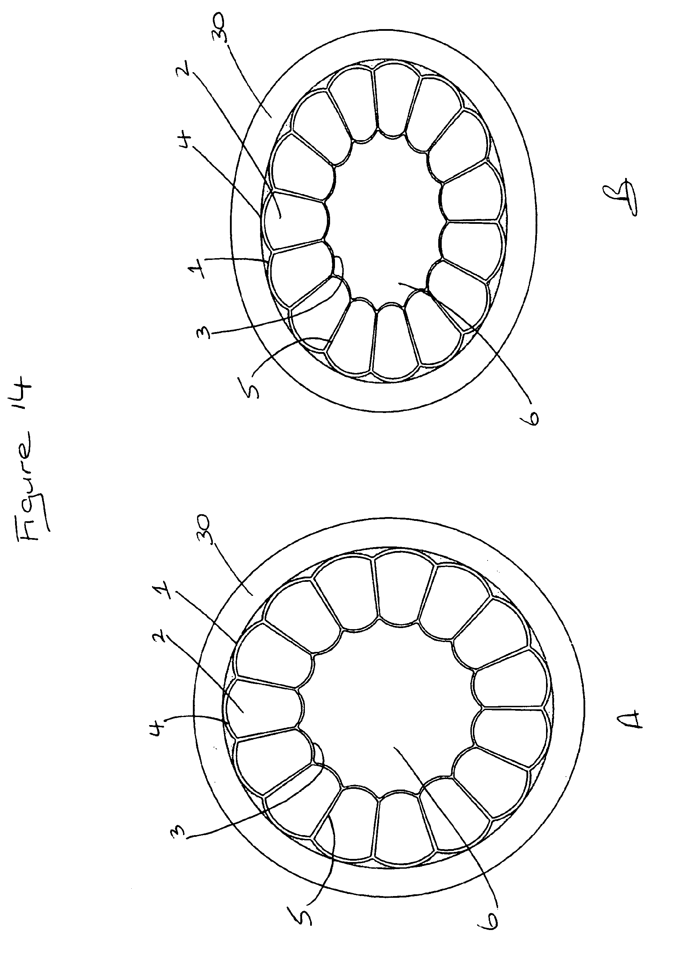

**Figures 14A and 14B** demonstrate the apposition of the inflatable device 1 of the present invention in those vessels which are substantially circular or non-circular in cross-sectional shape. The level of conformity achieved with the inflatable device 1 of the present invention is exemplary, particularly in non-circular shaped vessels. In particular in Figure 14B it is shown that a device of the invention can provide very good vessel wall contact even in vessels that are non-circular for example substantially elliptical in cross section. The fact that the device of the invention comprises an inflated annular cylinder allows it to adapt to varying vessel shapes.

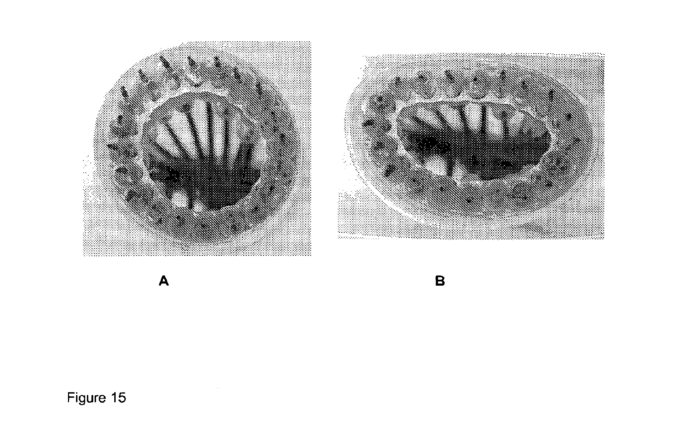

**Figures 15A and 15B** show photographic images of a device 1 of the invention that was constructed as follows: An elastic sheath with a number of lumen defined therein was moulded from silicone material. Non-compliant balloons were then inserted into the lumens.

As can be seen from Figures 15A and 15B, the device when moved to an inflated configuration conforms very well to the shape of the vessel in which it is place.

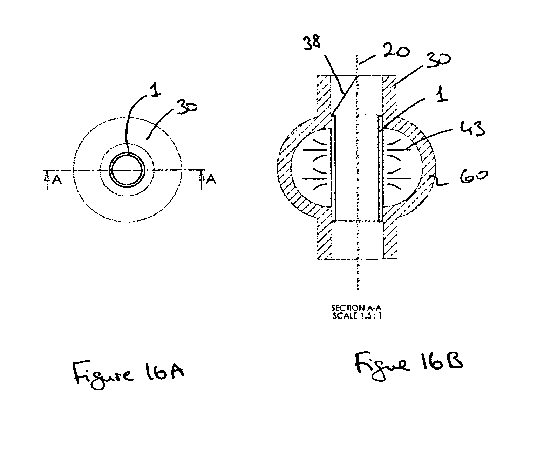

**Figures 16A and 16B** show respective cross-sectional views of a device 1 of the invention *in situ* within a vessel 30. The vessel 30 has a target site which is a bulging of the vessel caused by an aneurism 60. The device 1 is of the same construction as that shown in earlier Figures such as Figure 10. It is adapted to infuse a therapeutic material 43 which may be a clotting agent or other suitable agent for treating the aneurism. It will be appreciated that upon expansion (as shown in Figure 16B) the device 1 of the invention will abut and closely mate to the vessel 30. This means infused therapeutic material is protected from being carried away from the target site by blood flow. Residence time of the therapeutic material is increased thus also increasing the therapeutic effect.

It will be understood by those skilled in the art that any suitable compliant or non-compliant material may be chosen.

For example the non-compliant material, such as is suitable for forming a non-compliant balloon, is selected from (medical-grade) materials well known in the industry, for example, polyethylene, high-density polyethylene, polyethylene terephthalate, polyethylene block amide, polymethylacrylate, polypropylene, polytetrafluoroethylene, expanded polytetrafluoroethylene (ePTFE), polyimide, nylon (polyamide), polyacrylamide, polycarbonate, polyformaldehyde, polyvinylchloride, polyurethane, and the like, and combinations thereof. For example for compliance (medical-grade) compliant materials are also selected from those well known in the industry, for example, latex (natural rubber), silicone, polyurethane and combinations thereof.

It will be further understood by those skilled in the art that the inflatable device 1 can be implanted permanently or temporarily, depending on the modus operandi of the device, that is, whether the device is required to be *in situ* for a prolonged period of time or a short period of time. Further, the inflatable device 1 can also include bioresorbable materials such as poly(amino acids), poly(anhydrides), poly(caprolactones), poly(lactic/glycolic acid) polymers, poly(hydroxybutyrates) and poly(orthoesters).

In a further embodiment of the present invention, the inflatable device 1 can also comprise a radiopaque material to allow the device 1 to be tracked when deployed during an radiological intervention, and the like. Alternatively chemical sensors or physical detectors may form part of the device. For example fluoroscopy or ultrasound may be employed.

A device of the invention may be coated or impregnated with a therapeutic material such as an anticoagulant or the like. Where a filter attachment is present it may be desirable for the filter attachment to be additionally or alternatively coated or impregnated with a therapeutic material such as an anticoagulant or the like. Debris caught by the device can be removed by suction or physically broken-up.

The words "comprises/comprising" and the words "having/including" when used herein with reference to the present invention are used to specify the presence of stated features, integers, steps or components but do not preclude the presence or addition of one or more other features, integers, steps, components or groups thereof.

## Claims

1. An inflatable device (1) for use within the vasculature of a body and having an expandable annular body (1a) which is expandable by inflation of a series of inflation lumen (2) defined therein, the device having inflated and non-inflated states and inner (3) and outer (4) annular walls, the device being adapted so that inflationary pressure within the lumen moves the device from the non-inflated state toward the inflated state by radially outward expansion of both the outer and inner walls so that the annular body expands to form an annular structure with a central lumen (6) defined by the inner wall **characterized in that** the device comprises balloons (36) which occupy the lumens in the annular body.

2. A device according to Claim 1 comprising at least three, preferably at least four, inflation lumen (2); or
wherein the inner wall has a series of rebates (11) defined therein.

3. A device according to any preceding claim wherein the expandable annular body (1 a) is an annular double-walled balloon comprising:
(i) inner (3) and outer (4) annular balloon walls spaced apart from each other;
(ii) a series of inflation lumen (2) defined between said inner and outer walls.

4. A device according to any preceding claim wherein said inflation lumen are defined by partition walls (5) joining the inner and outer walls and further optionally wherein:
the partition walls (5) are constructed of a material that is more resilient to stretching than a material from which the internal and/or external wall is constructed; or
at least five partition walls are provided each joining the internal wall (4) to the external (5) wall.

5. A device according to any preceding claim wherein at least one, and desirably each, inflation lumen (2) narrows in a direction radially inwardly from the outer wall toward the inner wall, and further optionally wherein:
at least one, and desirably each, lumen (2) has in cross-section, a shape which is substantially that of a drop.

6. A device according to any preceding claim wherein said inflation lumens (2) are
defined by a surface having folds therein and optionally wherein
the inflation lumens have a substantially polygram shape in cross-section for example a polygram selected from the group consisting of those in the range from substantially pentagram to substantially hendecagram.

7. A device according to any preceding claim wherein the inflation lumen (2) are in
fluid communication with each other so that they may be inflated together; and/or wherein the inflation lumen are not in fluid communication with each other and are independently inflatable: and/or
further comprising at least one non-inflation lumen (10,12) defined within the body.

8. A device according to any preceding claim wherein in the inflated state the central lumen (6) has a diameter of at least about 1 mm, for example at least about 1.5 mm, preferably at least about 2.0mm, such as at least about 2.5 mm, for example at least about 3.0 mm, desirably at least about 3.5 mm.

9. A device according to any preceding claim wherein:
in the non-inflated state the body (1a) is stretch-fitted over a delivery
device; or
wherein the annular body (1a) is tethered distally to a guide wire (20); or
wherein the annular body (1a) is tethered proximally to a guide wire (20); or
wherein the annular body (1a) is tethered at proximal and distal ends to a guide wire, or optionally further comprising a mechanism to stretch the annular body in a substantially axial direction.

10. A device according to any preceding claim wherein the annular body (1a) is
constructed of a compliant material, and/or wherein the inflation lumen (2) comprise non-compliant balloons (36).

11. A device according to any preceding claim wherein the annular body (1a) is provided by an annular sheath of elastic material with a series of lumen defined therein, further optionally wherein the lumen in the annular body each house a non-compliant balloon (36).

12. A device according to any preceding claim further comprising a filter (22) which
extends across the central lumen (6) and which can act as an intraluminal embolic capture device to capture embolic material from blood passing through the central lumen (6); or
wherein the filter (22) is attached to a distal end of the annular body; or
wherein the filter (22) is attached to a proximal end of the annular body; or
wherein the annular body (1a) extends over a substantial portion of the length of the filter.

13. A device according to any preceding claim further comprising infusion means (41)
for infusing a substance into a vessel (30) and further optionally wherein
the device is adapted to infuse the substance when a predetermined inflated pressure in the annular body is reached, for example wherein the device comprises one or more rupturable reservoirs (41) which house the substance to be released and wherein the reservoirs are ruptured in use to release the substance.

14. A device according to Claim 13, wherein the device comprises infusion tubes on
the annular body; further optionally
wherein the infusion tubes are held to the annular body by elastic cuffs (50).

15. A device according to Claim 14, wherein the device further comprises a perforated sheath (51) optionally of elastic material enveloping the circumference of the annular body (1 a) and the infusion tubes or
wherein the device further comprises a porous sheath (55) enveloping the circumference of the annular body and the infusion tubes; or
wherein the sheath is bonded to a proximal and distal circumference of the annular body.

## Patentansprüche

1. Eine aufblasbare Vorrichtung (1) zur Verwendung innerhalb des Gefäßsystems eines Körpers und mit einem expandierbaren bzw. ausdehnbaren ringförmigen Körper (1 a), welcher durch das Aufblasen einer Reihe von Aufblashohlräumen bzw. Aufblaslumen (2), die darin definiert sind, ausdehnbar ist, wobei die Vorrichtung aufgeblasene und nicht aufgeblasene Zustände und innere (3) und äußere (4) ringförmige Wände hat, wobei die Vorrichtung so angepasst ist, dass der Inflations- bzw. Aufblasdruck innerhalb des Lumens die Vorrichtung von dem nicht aufgeblasenen Zustand zu dem aufgeblasenen Zustand hin bewegt, und zwar durch eine radial nach außen gerichtete Ausdehnung sowohl der äußeren als auch der inneren Wände, so dass sich der ringförmige Körper ausdehnt, um eine ringförmige Struktur mit einem zentralen Hohlraum bzw. Lumen (6) zu bilden, welcher durch die Innenwand definiert ist und **dadurch gekennzeichnet, dass** die Vorrichtung Ballons (36) aufweist, welche die Lumen in dem ringförmigen Körper besetzen bzw. einnehmen.

2. Vorrichtung nach Anspruch 1, welche mindestens drei, vorzugsweise mindestens vier Aufblaslumen (2) aufweist, oder
in der die Innenwand eine Reihe darin definierter Falze (11) besitzt.

3. Vorrichtung gemäß irgendeinem vorherigen Anspruch, wobei der ausdehnbare ringförmige Körper (1 a) ein ringförmiger doppelwandiger Ballon ist, welcher Folgendes aufweist:
(i) innere (3) und äußere (4) ringförmige Ballonwände, die voneinander beabstandet sind;
(ii) eine Reihe von Inflations- bzw. Aufblaslumen (2), die zwischen den inneren und äußeren Wänden definiert sind.

4. Vorrichtung gemäß irgendeinem vorherigen Anspruch, wobei die Aufblaslumen durch Trennwände (5), die die inneren und äußeren Wände verbinden, definiert werden, und wobei ferner optional die Trennwände (5) aus einem Material hergestellt sind, welches für Dehnung belastbarer ist als ein Material, aus dem die innere und/oder äußere Wand konstruiert ist; oder
wobei mindestens fünf Trennwände vorgesehen sind, wobei jede die Innenwand (4) mit der Außenwand (5) verbinden

5. Vorrichtung gemäß irgendeinem vorhergehenden Anspruch, wobei sich mindestens ein, und wünschenswerterweise jedes, Aufblaslumen (2) in einer Richtung radial nach innen von der Außenwand zur Innenwand hin verengt, und wobei ferner optional
mindestens ein, und vorzugsweise jedes, Lumen (2) im Querschnitt eine Form hat, die im Wesentlichen tropfenförmig ist.

6. Vorrichtung nach irgendeinem vorhergehenden Anspruch, wobei die Aufblaslumen (2) durch eine Oberfläche definiert werden, welche Falten darin besitzt und optional wobei
die Aufblaslumen im Querschnitt eine im Wesentlichen polygonale Form besitzen, beispielsweise ein Polygramm, welches aus der Gruppe ausgewählt ist, die aus jenen besteht im Bereich von im Wesentlichen einem Pentagramm bzw. Fünfeck bis im Wesentlichen einem Hendekagramm bzw. Elfeck.

7. Vorrichtung nach irgendeinem vorhergehenden Anspruch, wobei die Aufblaslumen (2) in Strömungsmittelverbindung miteinander stehen, so dass sie zusammen aufgeblasen werden können; und/oder wobei die Aufblaslumen nicht miteinander in Strömungsmittelverbindung stehen und unabhängig voneinander aufblasbar sind; und/oder welche ferner mindestens ein Nicht-Aufblasvolumen (10, 12) aufweist, welches innerhalb des Körpers definiert ist.

8. Vorrichtung nach irgendeinem vorhergehenden Anspruch, wobei das zentrale Lumen (6) in dem aufgeblasenen Zustand einen Durchmesser von mindestens etwa 1 mm besitzt, beispielsweise mindestens etwa 1,5 mm, vorzugsweise mindestens etwa 2,0 mm, wie etwa mindestens etwa 2,5 mm, zum Beispiel mindesten etwa 3,0 mm, wünschenswerterweise mindestens etwa 3,5 mm.

9. Vorrichtung nach irgendeinem vorhergehenden Anspruch, wobei in dem nicht aufgeblasenen Zustand der Körper (1 a) über eine Zuliefervorrichtung stretch- bzw. dehngepasst ist; oder
wobei der ringförmige Körper (1 a) distal an einen Führungsdraht (20) angebunden ist; oder
wobei der ringförmige Körper (1 a) proximal an einen Führungsdraht (20) angebunden ist; oder
wobei der ringförmige Körper (1 a) an proximalen und distalen Enden an einen Führungsdraht angebunden ist
oder ferner optional einen Mechanismus aufweist, um den ringförmigen Körper in einer im Wesentlichen axialen Richtung zu strecken bzw. dehnen.

10. Vorrichtung nach irgendeinem vorhergehenden Anspruch, wobei der ringförmige Körper (1 a) aus einem nachgiebigen Material konstruiert ist und/oder wobei die Aufblaslumen (2) nicht nachgiebige Ballons (36) aufweisen.

11. Vorrichtung nach irgendeinem vorhergehenden Anspruch, wobei der ringförmige Körper (1 a) durch eine ringförmige Umhüllung aus elastischem Material mit einer Reihe darin definierter Hohlräume bzw. Lumen vorgesehen ist, und ferner optional wobei die Lumen in dem ringförmigen Körper jeweils einen nicht nachgiebigen Ballon (36) aufnehmen.

12. Vorrichtung nach irgendeinem vorhergehenden Anspruch, welche ferner einen Filter (22) aufweist, welcher sich über das zentrale Lumen (6) erstreckt und welcher als eine intraluminale embolische Auffangvorrichtung wirken kann, um embolisches Material aus dem Blut, welches durch das zentrale Lumen (6) fließt, aufzufangen; oder
wobei der Filter (22) an einem distalen Ende des ringförmigen Körpers angebracht ist; oder
wobei der Filter (22) an einem proximalen Ende des ringförmigen Körpers angebracht ist; oder
wobei sich der ringförmige Körper (1 a) über einen wesentlichen Teil der Länge des Filters erstreckt.

13. Vorrichtung nach irgendeinem vorhergehenden Anspruch, welche ferner Infusionsmittel (41) zum Infundieren einer Substanz in ein Blutgefäß (30) aufweist und ferner optional,
wobei die Vorrichtung geeignet ist, die Substanz zu infundieren, wenn ein vorbestimmter Inflationsdruck bzw. Aufblasdruck in dem ringförmigen Körper erreicht ist, beispielsweise wobei die Vorrichtung ein oder mehrere reißbare Reservoirs bzw. Behälter (41) aufweist, welche die Substanz, welche freigegeben werden soll, aufnimmt, und wobei die Behälter beim Gebrauch aufgerissen werden, um die Substanz freizugeben.

14. Vorrichtung nach Anspruch 13, wobei die Vorrichtung Infusionsleitungen bzw. Infusionsschläuche an dem ringförmigen Körper aufweist, ferner optional
wobei die Infusionsschläuche durch elastische Manschetten (50) an dem ringförmigen Körper gehalten werden.

15. Vorrichtung nach Anspruch 14, wobei die Vorrichtung ferner eine perforierte Umhüllung (51) aufweist, optional aus elastischem Material, welche den Umfang des ringförmigen Körpers (1a) und die Infusionsschläuche umgibt; oder
wobei die Vorrichtung ferner eine poröse Umhüllung (55) aufweist, die den Umfang des ringförmigen Körpers und die Infusionsschläuche umgibt; oder
wobei die Umhüllung mit einem proximalen und distalen Umfang des ringförmigen Körpers verbunden bzw. verklebt ist.

## Revendications

1. Dispositif gonflable (1) pour utilisation à l'intérieur du système vasculaire d'un corps et comprenant un corps annulaire expansible (1a), qui est expansible par gonflage d'une série de lumières de gonflage (2) définies à l'intérieur, le dispositif présentant des états gonflé et non gonflé et des parois annulaires interne (3) et externe (4), le dispositif étant conçu de façon que, sous l'effet de la pression de gonflage à l'intérieur de la lumière, le dispositif passe de l'état non-gonflé à l'état gonflé par expansion radiale vers l'extérieur des deux parois externe et interne de sorte que le corps annulaire s'expand pour former une structure annulaire pourvue d'une lumière centrale (6) définie par la paroi interne, **caractérisé en ce que** le dispositif comprend des ballonnets (36) qui occupent les lumières dans le corps annulaire.

2. Dispositif selon la revendication 1, comprenant au moins trois, de préférence au moins quatre lumières de gonflage (2); ou
dans lequel la paroi interne comporte une série de rabats (11) définis à l'intérieur.

3. Dispositif selon l'une quelconque des revendications précédentes, dans lequel le corps annulaire expansible (1a) est un ballonnet annulaire à double paroi, comprenant :
(i) des parois de ballonnet annulaire interne (3) et externe (4) espacées l'une de l'autre;
(ii) une série de lumières de gonflage (2) définies entre lesdites parois interne et externe.

4. Dispositif selon l'une quelconque des revendications précédentes, dans lequel lesdites lumières de gonflage sont définies par des parois de séparation (5) reliant les parois interne et externe et, en outre, dans lequel, éventuellement :
les parois de séparation (5) sont réalisées dans un matériau qui est plus résistant à l'étirement qu'un matériau dans lequel est réalisée la paroi interne et/ou externe; ou
au moins cinq parois de séparation sont prévues, chacune reliant la paroi interne (4) à la paroi externe (5).

5. Dispositif selon l'une quelconque des revendications précédentes, dans lequel au moins une, et, de préférence, chaque lumière de gonflage (2) s'effile radialement vers l'intérieur depuis la paroi externe vers la paroi interne, et en outre, dans lequel, éventuellement :
au moins une, et, de préférence, chaque lumière (2) présente une section transversale sensiblement en forme de goutte.

6. Dispositif selon l'une quelconque des revendications précédentes, dans lequel lesdites lumières de gonflage (2) sont définies par une surface pourvue de plis à l'intérieur et, éventuellement, dans lequel
les lumières de gonflage ont une section transversale sensiblement en forme de polygramme, par exemple un polygramme sélectionné dans le groupe comprenant ceux de la gamme allant de sensiblement en forme de pentagramme à sensiblement en forme d'hendécagramme.

7. Dispositif selon l'une quelconque des revendications précédentes, dans lequel les lumières de gonflage (2) sont en communication fluidique entre elles de façon à pouvoir être gonflées ensemble; et/ou dans lequel les lumières de gonflage ne sont pas en communication fluidique entre elles et sont gonflables de manière indépendante; et/ou
comprenant, en outre, au moins une lumière non gonflable (10, 12) définie à l'intérieur du corps.

8. Dispositif selon l'une quelconque des revendications précédentes, dans lequel, à l'état gonflé, la lumière centrale (6) a un diamètre d'au moins env. 1 mm, par exemple au moins env. 1,5 mm, de préférence au moins env. 2,0 mm, tel qu'au moins env. 2,5 mm, par exemple au moins env. 3,0 mm, préférablement au moins env. 3,5 mm.

9. Dispositif selon l'une quelconque des revendications précédentes, dans lequel
à l'état non gonflé, le corps (1a) est monté tendu sur un dispositif de délivrance; ou
dans lequel le corps annulaire (1a) est fixé de manière distale à un fil-guide (20); ou
dans lequel le corps annulaire (1a) est fixé de manière proximale à un fil-guide (20); ou
dans lequel le corps annulaire (1a) est fixé aux extrémités proximale et distale à un fil-guide, ou comprenant, en outre, éventuellement un mécanisme destiné à étirer le corps annulaire dans une direction sensiblement axiale.

10. Dispositif selon l'une quelconque des revendications précédentes, dans lequel le corps annulaire (1a) est réalisé dans un matériau élastique, et/ou dans lequel les lumières de gonflage comprennent des ballonnets (36) non élastiques.

11. Dispositif selon l'une quelconque des revendications précédentes, dans lequel le corps annulaire (1a) est pourvu d'une gaine annulaire en matériau élastique doté d'une série de lumières définies à l'intérieur, dans lequel, en outre, éventuellement, les lumières dans le corps annulaire reçoivent chacune un ballonnet (36) non élastique.

12. Dispositif selon l'une quelconque des revendications précédentes, comprenant, en outre, un filtre (22) qui s'étend à travers la lumière centrale (6) et qui peut servir de dispositif intraluminal de capture de matières emboliques pour capturer des matières emboliques dans le sang passant à travers la lumière centrale (6); ou
dans lequel le filtre (22) est fixé à une extrémité distale du corps annulaire; ou
dans lequel le filtre (22) est fixé à une extrémité proximale du corps annulaire; ou
dans lequel le corps annulaire (1a) s'étend sur une grande partie de la longueur du filtre.

13. Dispositif selon l'une quelconque des revendications précédentes, comprenant, en outre, des moyens de perfusion (41) pour perfuser une substance dans un vaisseau (30) et en outre, éventuellement, le dispositif étant conçu pour perfuser la substance lorsqu'une pression de gonflage prédéterminée dans le corps annulaire est atteinte, par exemple, le dispositif comprenant un ou plusieurs réservoirs rupturables (41) qui reçoivent la substance à libérer et les réservoirs étant rompus en cours d'utilisation pour libérer la substance.

14. Dispositif selon la revendication 13, dans lequel le dispositif comprend des tubes de perfusion sur le corps annulaire; en outre, éventuellement,
dans lequel les tubes de perfusion sont maintenus sur le corps annulaire par des manchettes élastiques (50).

15. Dispositif selon la revendication 14, dans lequel le dispositif comprend, en outre, une gaine perforée (51) éventuellement en matériau élastique enveloppant la circonférence du corps annulaire (1a) et les tubes de perfusion ou
dans lequel le dispositif comprend, en outre, une gaine poreuse (55) enveloppant la circonférence du corps annulaire et les tubes de perfusion; ou
dans lequel la gaine est liée à une circonférence proximale et distale du corps annulaire.
